# EUROPEAN PATENT APPLICATION

(11) **EP 3 792 345 A1**
(43) Date of publication of application: **17.03.2021**
(21) Application number: 19793551.3
(22) Date of filing: 25.04.2019
(51) Int. Cl.: C12N 5/071, C12N 5/02, C12N 5/0735

(54) **METHOD FOR PROMOTING DIFFERENTIATION OF PLURIPOTENT STEM CELLS**

(30) Priority: 26.04.2018 JP 2018085591; 12.12.2018 JP 2018232436
(71) Applicant: Tokyo Institute of Technology, Tokyo 152-8550 (JP); AJINOMOTO CO., INC., Chuo-ku Tokyo 104-8315 (JP)
(72) Inventor: KUME, Shoen, Tokyo 152-8550 (JP); SHIRAKI, Nobuaki, Tokyo 152-8550 (JP); CHIBA, Akira, Tokyo 104-8315 (JP); ENOMOTO, Takayuki, Tokyo 152-8550 (JP)
(74) Representative: J A Kemp LLP
(86) International application number: PCT/JP2019/017703
(87) International publication number: WO 2019/208713

(57) **Abstract**

In order to improve the efficiency of inducing differentiation of pluripotent stem cells, provided is a method for promoting differentiation of pluripotent stem cells, the method including a step of culturing pluripotent stem cells in a medium, wherein the medium is a medium containing a) an insulin-like growth factor, b) an insulin analogue preparation containing no zinc, c) an insulin analogue preparation containing a low concentration of zinc, or d) a compound exhibiting an insulin-like action.

## Description

### Technical Field

The present invention relates to a method for promoting differentiation of pluripotent stem cells, a medium for culturing pluripotent stem cells, and a method for inducing differentiation into insulin-producing cells.

### Background Art

Due to their pluripotency, embryonic stem cells (ES cells) and induced pluripotent stem cells (iPS cells) are useful models for studying gene functions during development and are also expected to be applied to regenerative medicine since they may serve as transplantable cell sources for medical care. However, for example, in order to use ES cells and iPS cells for cell replacement therapy and tissue transplantation in diseases such as diabetes, it is necessary to control differentiation.

Therefore, many studies have been actively conducted in recent years to control the differentiation from undifferentiated cells into tissue cells. For example, in-vitro production of endoderm cells and insulin-producing cells from mouse and human ES cells has been reported (Kubo A et al. Development, 131: 1651-1662, 2004, D'Amour KA et al. Nat Biotechnol 24: 1392-1401, 2006) .

Further, there have been reported a method for efficiently inducing differentiation of ES cells into pancreatic progenitor cells by using mouse mesonephric cell line M15 cells as support cells and adding activin, an FGF (fibroblast growth factor), and retinoic acid to a medium (International Publication No. WO 2006/126574, Shiraki, N. et al. Stem Cells, 26: 874-885, 2008), and a method for efficiently inducing differentiation of mouse and human ES cells into liver cells using mmcM15 cells as support cells in culture conditions of adding or removing specific secretory growth factors (such as an FGF and BMP (Bone Morphogenetic Proteins)) (International Publication No. WO 2008/149807, Yoshida T. et al. Genes Cells, 13: 667-678, 2008).

However, in the case of inducing differentiation of ES cells and iPS cells into various cells, undifferentiated stem cells remain and are partially incorporated. In regenerative medicine, there are concerns about the safety, such as the possibility that such cells may become cancerous. Thus, there has been a need for a technique for increasing the efficiency of inducing differentiation and preventing incorporation of undifferentiated cells.

Under such a background, the inventor has conducted research on a method for promoting induction of differentiation from pluripotent stem cells into digestive organ cells such as pancreas, liver, and intestine, thereby finding that differentiation of pluripotent stem cells can be promoted by culturing the cells in a medium with methionine deprived, and thus has filed a patent application previously (Patent Literature 1) .

### Citation List

### Patent Literature

Patent Literature 1: International Publication No. WO 2015/123662

### Summary of Invention

### Technical Problem

The undifferentiated stem cells remaining in the induction of differentiation of pluripotent stem cells are concerns in ensuring the safety of regenerative medicine, as described above.

The present invention has been made under such a background, and an object of the present invention is to provide a new method for improving the efficiency of inducing differentiation of pluripotent stem cells, to solve the problem of undifferentiated stem cells remaining in the induction of differentiation.

### Solution to Problem

The inventor conducted analysis focusing on the kinetics of intracellular zinc generated by methionine deprival in the process of clarifying the mechanism of action to promote the differentiation by methionine deprival. As a result, it was found that intracellular zinc has an action to promote the growth of undifferentiated cells and exhibits an action to inhibit the differentiation.

Meanwhile, insulin is generally added as a growth factor in the induction of differentiation of pluripotent stem cells into cells such as insulin-positive cells. In general, zinc is present in the nucleus of insulin crystals forming a hexamer. Accordingly, zinc is contained in a medium when an insulin preparation forming a hexamer is added. Therefore, the inventor devised and actually tried a method of adding an insulin-like growth factor (IGF) instead of insulin as a method for preventing incorporation of zinc in the medium and inhibition of cell proliferation. As a result, it has been found that the efficiency of inducing differentiation into endoderm has increased significantly in a medium using IGF.

The present invention has been accomplished based on the aforementioned findings.

That is, the present invention provides [1] to [18] below.
[1] A method for promoting differentiation of pluripotent stem cells, comprising a step of culturing pluripotent stem cells in a medium, wherein the medium is a medium comprising a) an insulin-like growth factor, b) an insulin analogue preparation comprising no zinc, c) an insulin analogue preparation comprising a low concentration of zinc, or d) a compound exhibiting an insulin-like action.
[2] The method for promoting differentiation of pluripotent stem cells according to [1], wherein the medium for culturing pluripotent stem cells is a medium comprising no insulin.
[3] The method for promoting differentiation of pluripotent stem cells according to [1] or [2], wherein the medium for culturing pluripotent stem cells is a medium comprising zinc at a concentration of 1 µM or less or a medium comprising no zinc.
[4] The method for promoting differentiation of pluripotent stem cells according to any one of [1] to [3], wherein the medium for culturing pluripotent stem cells is a medium allowing the pluripotent stem cells to differentiate into endoderm cells.
[5] The method for promoting differentiation of pluripotent stem cells according to any one of [1] to [4], comprising a step of culturing pluripotent stem cells in an undifferentiated maintenance medium, wherein the undifferentiated maintenance medium is a medium comprising no methionine.
[6] The method for promoting differentiation of pluripotent stem cells according to [1], wherein the medium for culturing pluripotent stem cells is a medium comprising an activin receptor-like kinase-4,7 activator and comprising zinc at a concentration of 1 µM or less or no zinc.
[7] The method for promoting differentiation of pluripotent stem cells according to [6], wherein the activin receptor-like kinase-4,7 activator is human activin A, and a concentration thereof is 6 to 150 ng/mL.
[8] A medium for culturing pluripotent stem cells, comprising a) an insulin-like growth factor, b) an insulin analogue preparation comprising no zinc, c) an insulin analogue preparation comprising a low concentration of zinc, or d) a compound exhibiting an insulin-like action.
[9] The medium according to [8], comprising no insulin.
[10] The medium according to [8] or [9], comprising zinc at a concentration of 1 µM or less or no zinc.
[11] The medium according to any one of [8] to [10], wherein the medium is a medium allowing the pluripotent stem cells to differentiate into endoderm cells.
[12] The medium according to [8], wherein the medium for culturing pluripotent stem cells is a medium comprising an activin receptor-like kinase-4,7 activator and comprising zinc at a concentration of 1 µM or less or no zinc.
[13] The medium according to [12], wherein the activin receptor-like kinase-4,7 activator is human activin A, and a concentration thereof is 6 to 150 ng/mL.
[14] A method for inducing differentiation of pluripotent stem cells into insulin-producing cells, comprising steps (1) to (6) below:
   (1) a step of culturing pluripotent stem cells in a medium comprising no methionine;
   (2) a step of culturing the cells obtained in step (1) in the medium according to [12] or [13];
   (3) a step of culturing the cells obtained in step (2) in a medium comprising an FGF and a hedgehog signaling inhibitor;
   (4) a step of culturing the cells obtained in step (3) in a medium comprising a retinoic acid receptor agonist, a hedgehog signaling inhibitor, and a BMP signaling inhibitor;
   (5) a step of culturing the cells obtained in step (4) in a medium comprising a TGF-βI type activin receptor-like kinase-4,5,7 inhibitor, a protein kinase C activator, and a BMP signaling inhibitor; and
   (6) a step of culturing the cells obtained in step (5) in a medium comprising nicotinamide.
[15] The method for inducing differentiation into insulin-producing cells according to [14], wherein each medium in step (1) to (6) is a xeno-free medium.
[16] A method for inducing differentiation of pluripotent stem cells into insulin-producing cells, comprising steps (1) to (9), or (1) to (3) and (5) to (9) below:
   (1) a step of culturing pluripotent stem cells in a medium comprising no methionine;
   (2) a step of culturing the cells obtained in step (1) in the medium according to [12] or [13];
   (3) a step of culturing the cells obtained in step (2) in a medium comprising an FGF and a hedgehog signaling inhibitor;
   (4) a step of culturing the cells obtained in step (3) in a medium comprising a KGF;
   (5) a step of culturing the cells obtained in step (3) or step (4) in a medium comprising a KGF, a hedgehog signaling inhibitor, a protein kinase C activator, and a retinoic acid receptor agonist;
   (6) a step of culturing the cells obtained in step (5) in a medium comprising a KGF, a hedgehog signaling inhibitor, a retinoic acid receptor agonist, and a BMP signaling inhibitor;
   (7) a step of culturing the cells obtained in step (6) in a medium comprising a TGF-βI type activin receptor-like kinase-4,5,7 inhibitor, an EGF receptor agonist, a γ-secretase inhibitor, and a retinoic acid receptor agonist;
   (8) a step of culturing the cells obtained in step (7) in a medium comprising a TGF-βI type activin receptor-like kinase-4,5,7 inhibitor, an EGF receptor agonist, a γ-secretase inhibitor, and a retinoic acid receptor agonist, wherein a concentration of the retinoic acid receptor agonist in the medium is lower than a concentration of the retinoic acid receptor agonist in step (7); and
   (9) a step of culturing the cells obtained in step (8) in a medium comprising a TGF-βI type activin receptor-like kinase-4,5,7 inhibitor.
[17] A method for differentiating pancreatic progenitor cells into pancreatic beta cells, comprising culturing pancreatic progenitor cells in a medium comprising a factor selected from the group consisting of a retinoic acid receptor agonist, a hedgehog signaling inhibitor, a KGF, a γ-secretase inhibitor, a BMP signaling inhibitor, a TGF-βI type activin receptor-like kinase-4,5,7 inhibitor, and an EGF receptor agonist, wherein the pancreatic progenitor cells are PDX1 positive or NKX6.1 positive.
[18] A method for inducing differentiation of pluripotent stem cells into insulin-producing cells, comprising steps (1) to (7) below:
   (1) a step of culturing pluripotent stem cells in a medium comprising no methionine;
   (2) a step of culturing the cells obtained in step (1) in the medium according to [12] or [13];
   (3) a step of culturing the cells obtained in step (2) in a medium comprising a KGF and comprising zinc at a concentration of 1 µM or less or no zinc;
   (4) a step of culturing the cells obtained in step (3) in a medium comprising a KGF, a hedgehog signaling inhibitor, and a retinoic acid receptor agonist;
   (5) a step of culturing the cells obtained in step (4) in a medium comprising a KGF, a hedgehog signaling inhibitor, and a retinoic acid receptor agonist, wherein a concentration of the retinoic acid receptor agonist in the medium is lower than a concentration of the retinoic acid receptor agonist in step (4);
   (6) a step of culturing the cells obtained in step (5) in a medium comprising a TGF-βI type activin receptor-like kinase-4,5,7 inhibitor and a retinoic acid receptor agonist; and
   (7) a step of culturing the cells obtained in step (6) in a medium comprising a TGF-βI type activin receptor-like kinase-4,5,7 inhibitor and a retinoic acid receptor agonist, wherein a concentration of the retinoic acid receptor agonist in the medium is lower than a concentration of the retinoic acid receptor agonist in step (6).

This description includes the contents described in the description and/or drawings of Japanese patent applications (Japanese Patent Application No. 2018-085591 and Japanese Patent Application No. 2018-232436), which are the basis of the priority of this application.

### Advantageous Effects of Invention

The present invention provides a new method for promoting differentiation of pluripotent stem cells. The efficiency of inducing differentiation from pluripotent stem cells into cells of pancreas, liver, intestine, and the like, particularly, the efficiency of inducing differentiation in the initial stage of differentiation can be improved by this method.

### Brief Description of Drawings

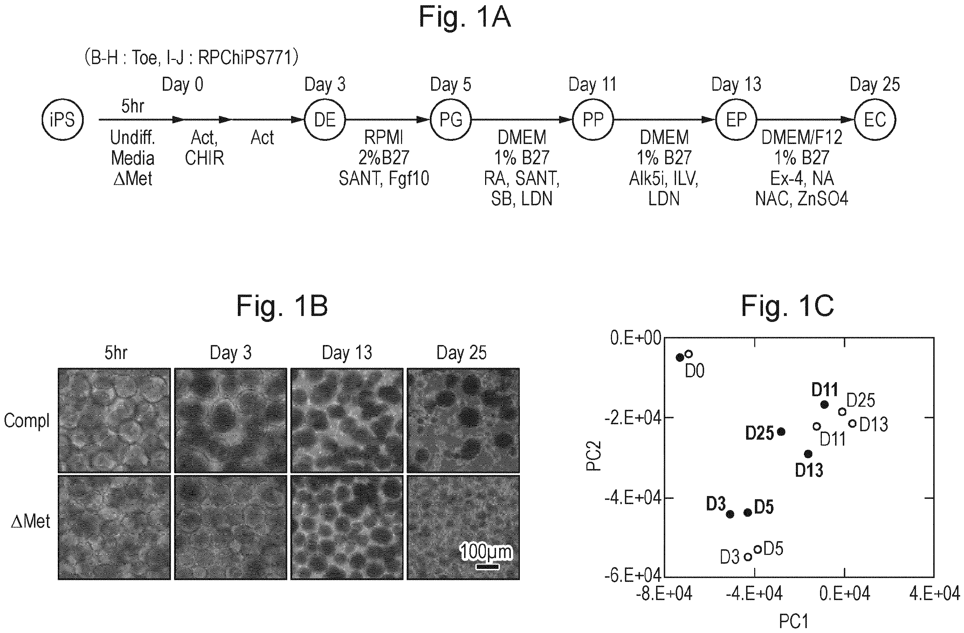

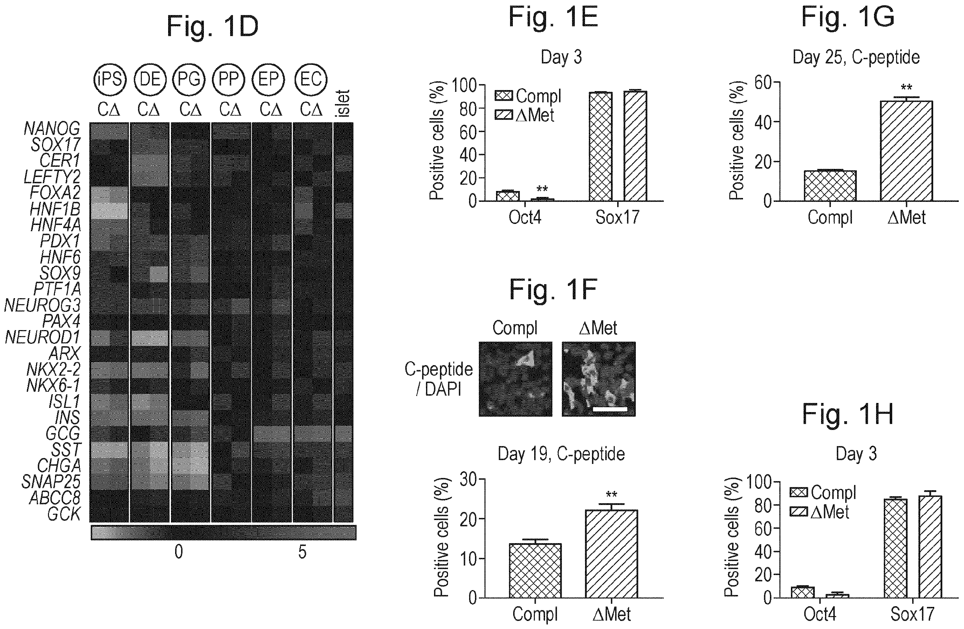

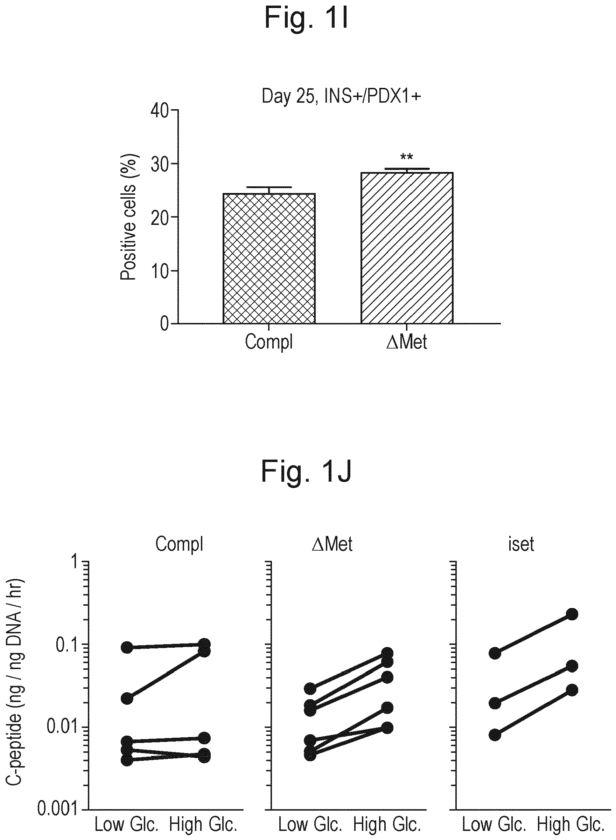

[Figure 1] Methionine deprival pretreatment potentiated late stage differentiation of human iPS cells into insulin-expressing beta cells. When pretreated with short term methionine deprival before differentiation, Toe and RPChiPS771 human iPS cells showed an increased differentiation potency and generated glucose responsive beta cells, with an activity resembling that of the human pancreatic islets. A) Schematic drawing of the differentiation procedure. Undifferentiated Toe (B-G) or RPChiPS771 (H-J) hiPSCs dissociated and cultured to form spheres, were treated with (or without) methionine deprival, then triggered to differentiate by switching to differentiation media, and followed the 5-step differentiation procedure for differentiation into pancreatic beta cells. B) Representative bright field images of the cells, immediately after methionine pretreatment (5h), and on differentiation days 3, 13, and 25 are shown. The upper panels show culture in a control complete medium, and the lower panels show culture in a methionine-deprived medium. C) Gene array analyses of the undifferentiated hiPSCs (D0), and differentiation day 3 DE, day 5 PG, day 11 PP, day 13 EP, and day 25 EC were analyzed by principal component analysis. D) The expression of maturation and differentiation related genes in the differentiated iPSCs are shown by a heat map. Results obtained with human pancreatic islets are also shown. E) Immunocytochemical analysis presented as OCT3/4 or SOX17 positivity on differentiation day 3. N = 3. F, G) Representative C-peptide positive staining images on differentiation day 19 (F, upper panel). Quantitative result of C-peptide positivity on differentiation day 19 (F, lower panel), or differentiation day 25 (G) are shown. N = 3. H-J) RPChiPS771 hiPSCs were also tested. H) RPChiPS771 hiPSCs showed significantly decreased OCT3/4 expression upon methionine deprival on differentiation day 3, although the increase in SOX17 positivity was not evident. N = 3. I) An increase in INS+/PDX1+ cells on differentiation day 25 was observed upon methionine deprival. N = 3. J) GSIS activity of the RPChiPS771 hiPSC-derived cells and human pancreatic islets. Human iPSC-derived beta cells pre-treated with methionine deprival (middle panel, n = 6) showed an improved glucose-stimulated C-peptide secretion compared to that of the control cells (left panel, Compl, n = 5), with a high to low glucose ratio that resembles that of human pancreatic islets (right panel, n = 3). Data are expressed as the mean ± SEM. Differences between groups were analyzed by Student's t-tests. The significance is shown as *p < 0.05 or **p < 0.01.

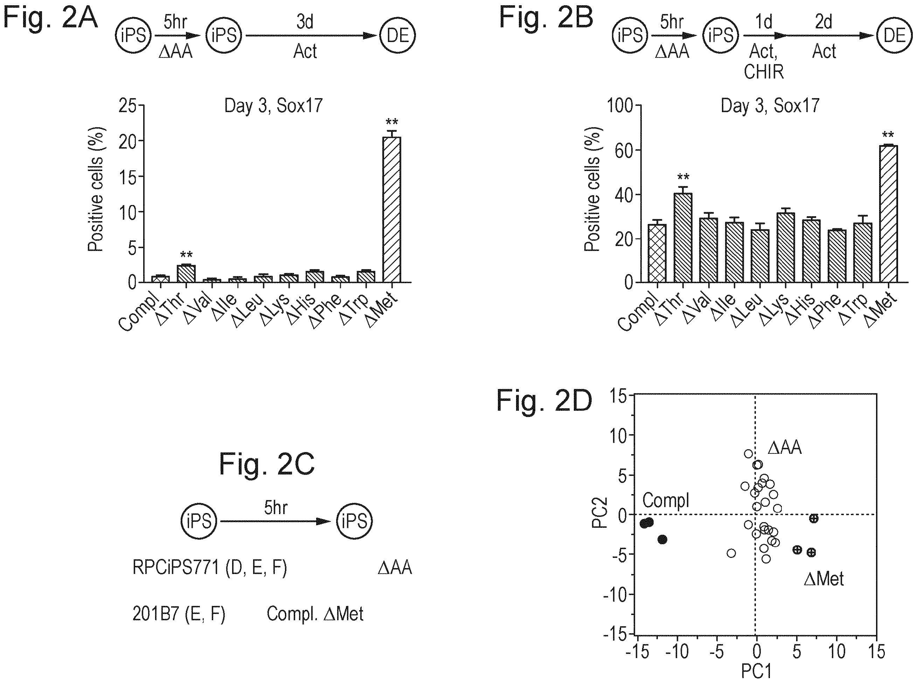

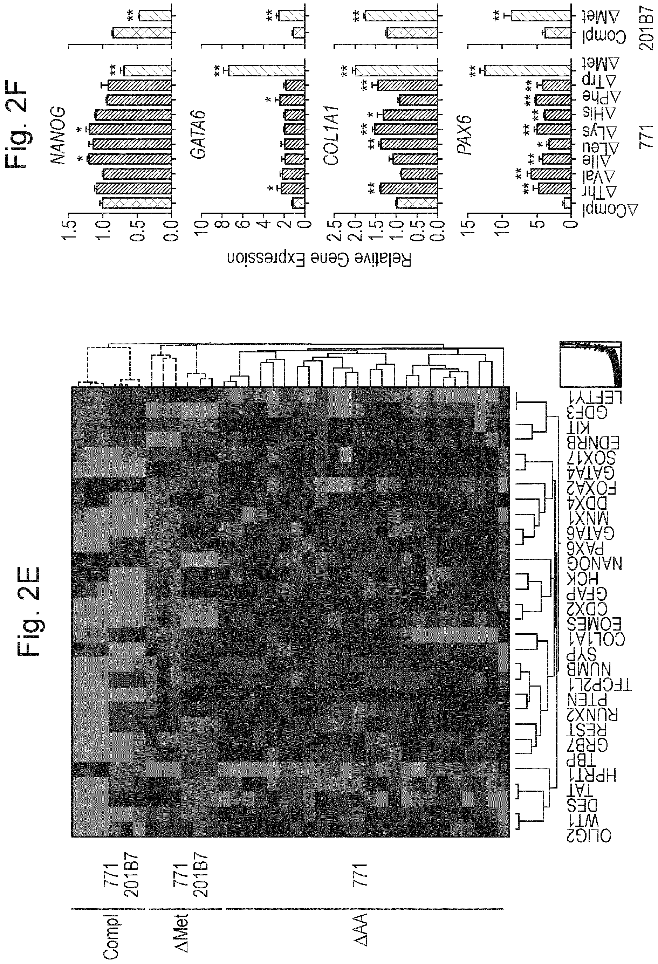

[Figure 2] Gene expression analysis of methionine deprival pretreated cells revealed downregulation of pluripotent cell marker, NANOG, and upregulation of differentiation related genes. A, B) Schematic drawings of differentiation procedures for differentiation into definitive endoderm. RPChiPS771 hiPSCs were pretreated with single amino acid deprival singly for 5 h, were then triggered to differentiate by switching to differentiation medium as shown in the schematic drawing under different conditions: (A) differentiation in medium 1 was done by adding Activin for 3 days (without CHIR99021) or (B) adding activin and CHIR99021 for 1 day and then without CHIR99021 but with Activin for 2 days. Under either protocol, potentiation of differentiation was observed in cells pretreated with Met or Thr deprival, but not other amino acid deprival. C) A schematic drawing of the experiment showing that undifferentiated RPChiPS771 or 201B7 hiPSCs were treated with control complete medium or methionine deprival for 5h. D) Methionine deprival treated or other amino acid singly deprival treated cells were subjected to gene expression analysis using a primer array set for embryonic stem cells. The results were compared using principal component analysis. E) The results are presented as a heat map. F) Genes, whose expressions were most affected by methionine deprival, are shown. NANOG, a pluripotency marker, whose expression is decreased by Met deprival. GATA6, C0L1A1 and PAX6, differentiation marker genes, whose expressions are increased by methionine deprival. Data are expressed as the mean ± SEM. N = 3. Differences between groups were analyzed by one-way ANOVA and Dunnett's multiple comparison test. Significance is shown as *P < 0.05 or **P < 0.01, by one-way ANOVA and Dunnett's multiple comparison test.

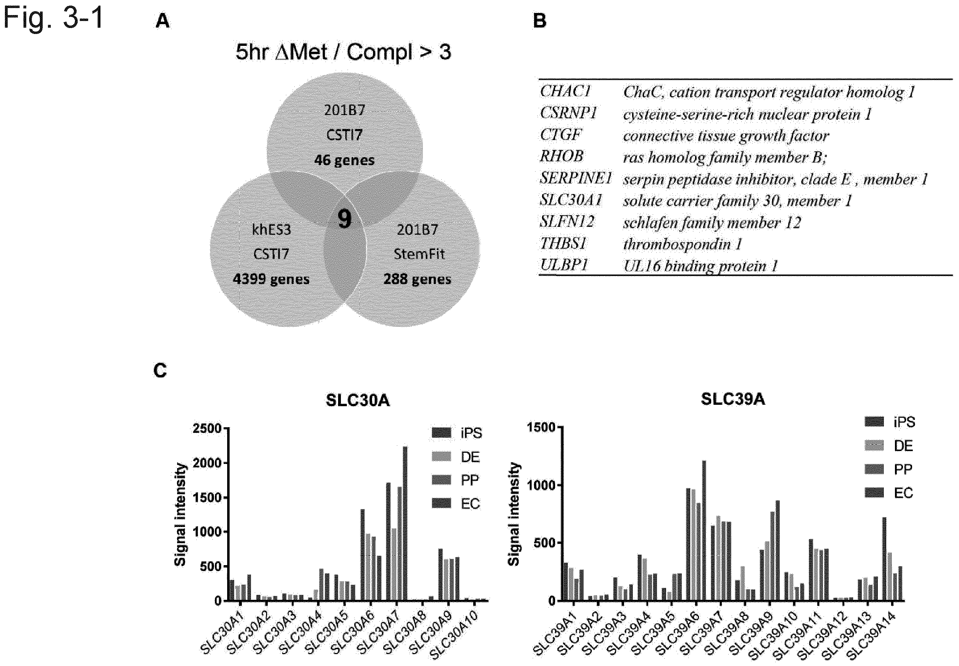

[Figure 3-1] Gene array analysis identified SLC30A1 as a gene whose expression increased specifically upon methionine deprival. A) Three experiments of gene expression array analyses of hiPSCs deprived with methionine or control complete medium were performed with hESC khES3, and hiPSC 201B7. Nine genes observed to be up-regulated in methionine deprived group were extracted, and listed in B. B) The results identified SLC30A1 as a gene increased specifically upon methionine deprival, together with other 8 genes. C) Expression of members of SLC30A and SLC39A family genes in the undifferentiated hiPSCs or hiPSC-derived differentiated pancreatic cells are extracted from the results of the gene array analyses by the inventor. Only SLC30A and SLC39A genes whose expression were detected are shown.

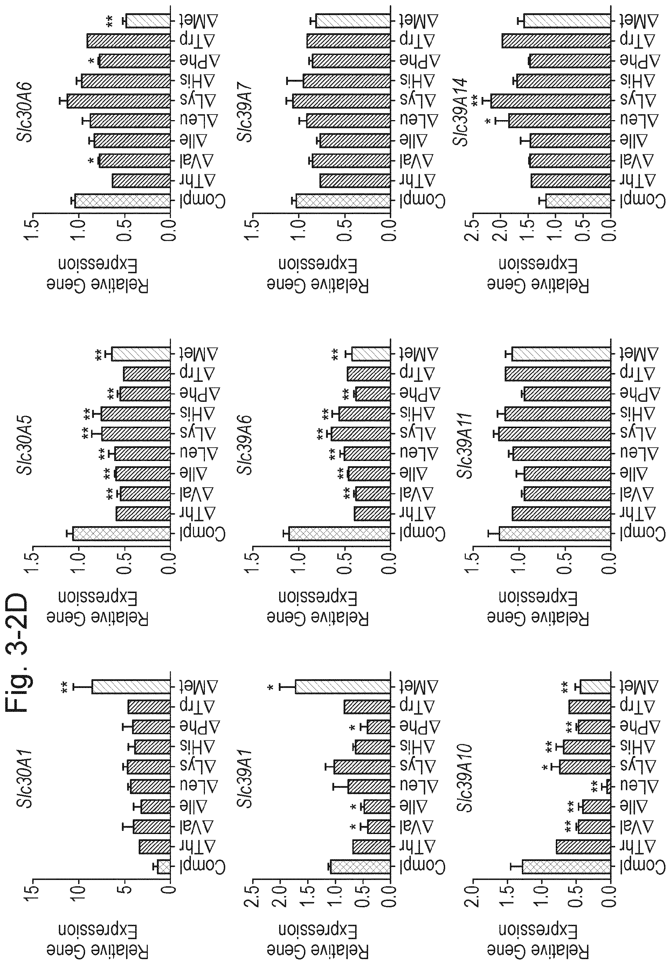

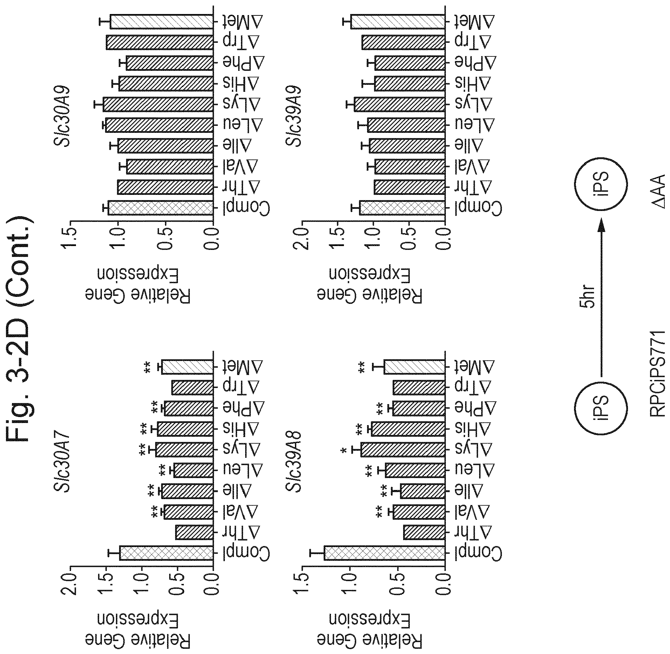

[Figure 3-2] Gene array analysis identified SLC30A1 as a gene whose expression increased specifically upon methionine deprival. D) Real time PCR analysis of the expression of SLC30A and SLC39A genes upon deprival of each amino acid deprival singly. Only SLC30A1, but not other genes showed specific changes of expression upon methionine deprival. N = 3, excluding ΔThr, ΔTrp, n = 2. Data are expressed as the mean ± SEM. Differences between groups were analyzed by one-way ANOVA and Dunnett's multiple comparisons test (C). Significance is shown as *P < 0.05 or **P < 0.01, by one-way ANOVA and Dunnett's multiple comparisons test.

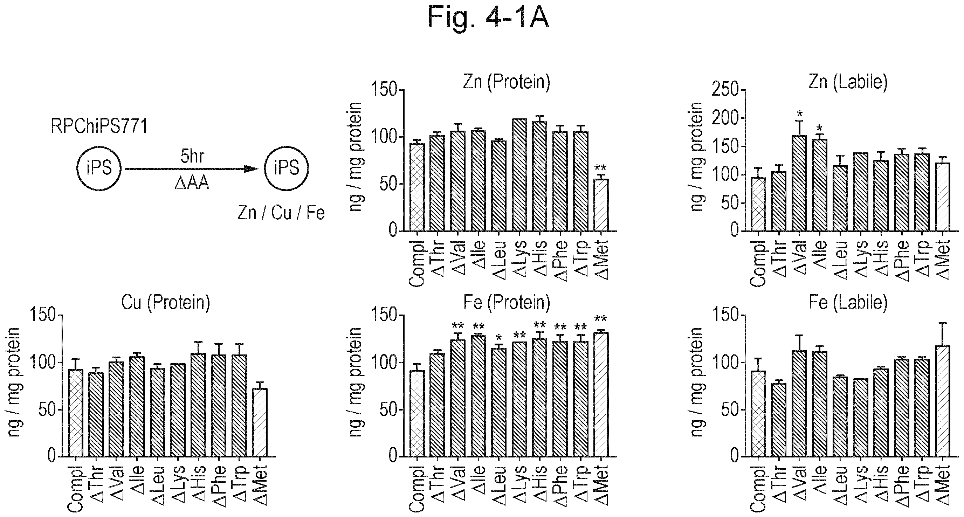

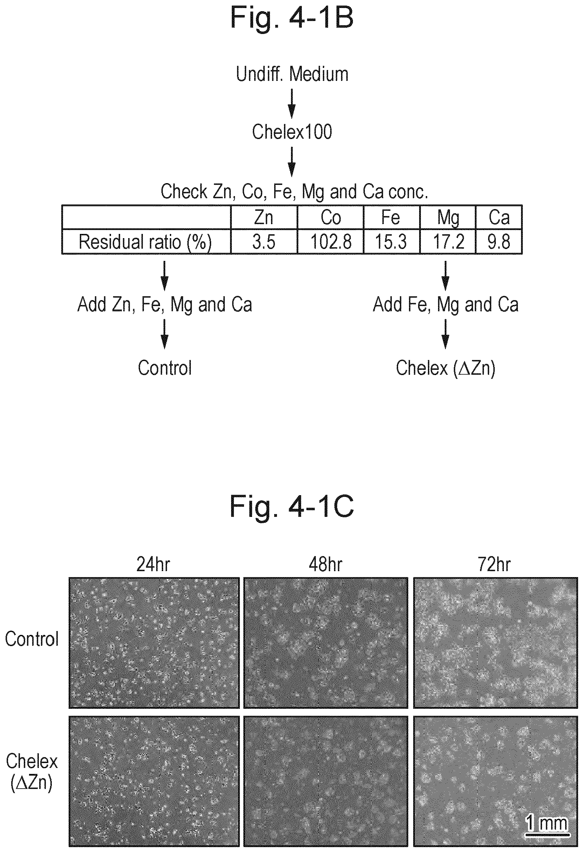

[Figure 4-1] Methionine deprival resulted in reduced intracellular protein bound Zn in iPSCs, which was mimicked by culturing iPSCs in Zn deprived medium. A) RPChiPS771 was treated with methionine deprival or other amino acids singly deprival for 5 h and subjected to analysis of heavy metals of Zn, Cu and Fe in the hiPSCs. Protein bound Zn significantly decreased in iPSC cultured under methionine deprival, but not other amino acids. Fe or Cu was not specifically affected by methionine deprival. (B) ΔZn undifferentiated medium was prepared using Chelex 100 treatment. Concentrations of heavy metals (Zn, Co, Fe, Mg and Ca) were measured and were added back to prepare ΔZn or control complete medium (Zn also added back). C) Bright field images of undifferentiated RPChiPS771 cells cultured with control complete medium or ΔZn medium for 24, 48 or 72 h are shown. Data are expressed as the mean ± SEM. N = 3. Differences between groups were analyzed by Student's t-test. Significance is shown as * p < 0.05 or ** p < 0.01.

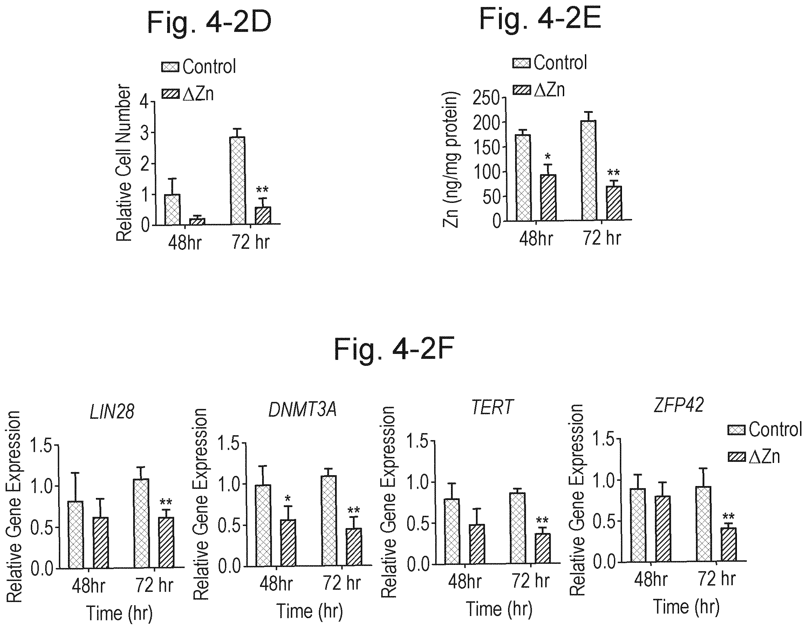

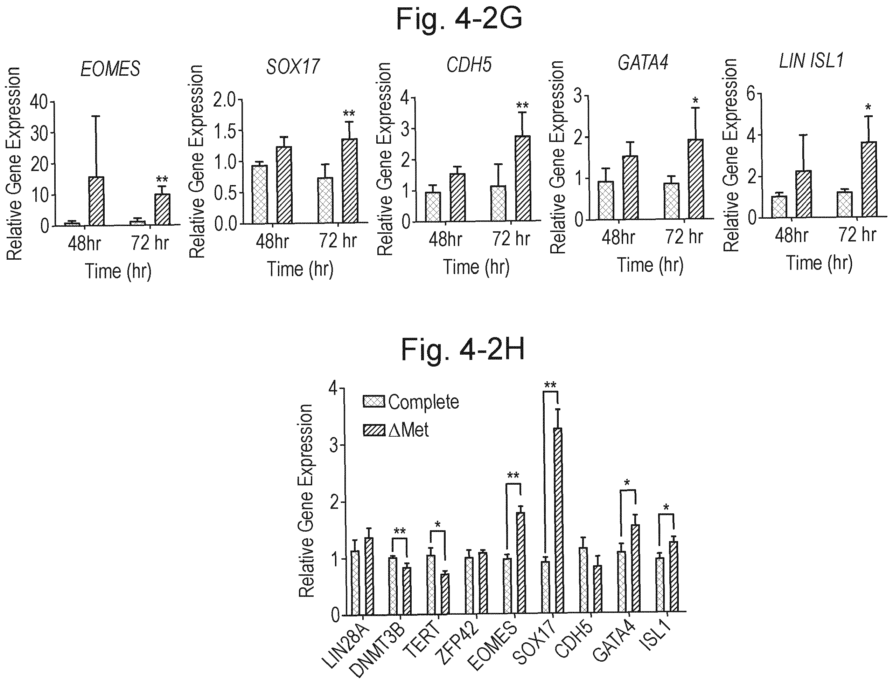

[Figure 4-2] Methionine deprival resulted in reduced intracellular protein bound Zn in iPSCs, which was mimicked by culturing iPSCs in Zn deprived medium. D) Relative cell number, (E) intracellular Zn concentration decreased with increasing time cultured under ΔZn. (F, G) Expressions of gene markers for pluripotency and proliferation (F) or differentiation (G) are shown. (H) Expressions of gene markers were examined with cells treated with methionine deprival. Data are expressed as the mean ± SEM. N = 3. Differences between groups were analyzed by Student's t-test. Significance is shown as * p < 0.05 or ** p < 0.01.

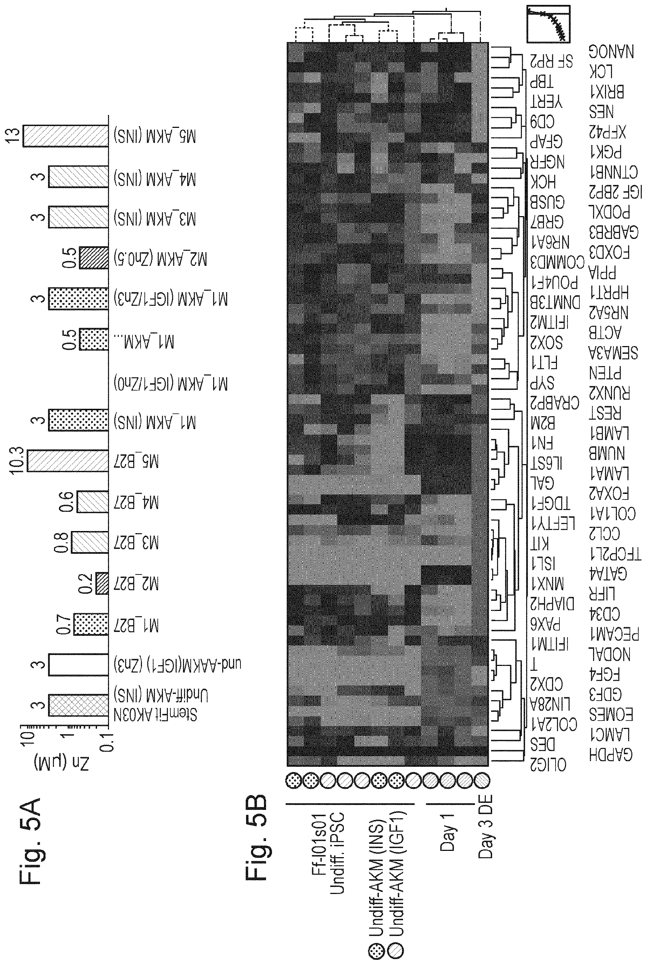

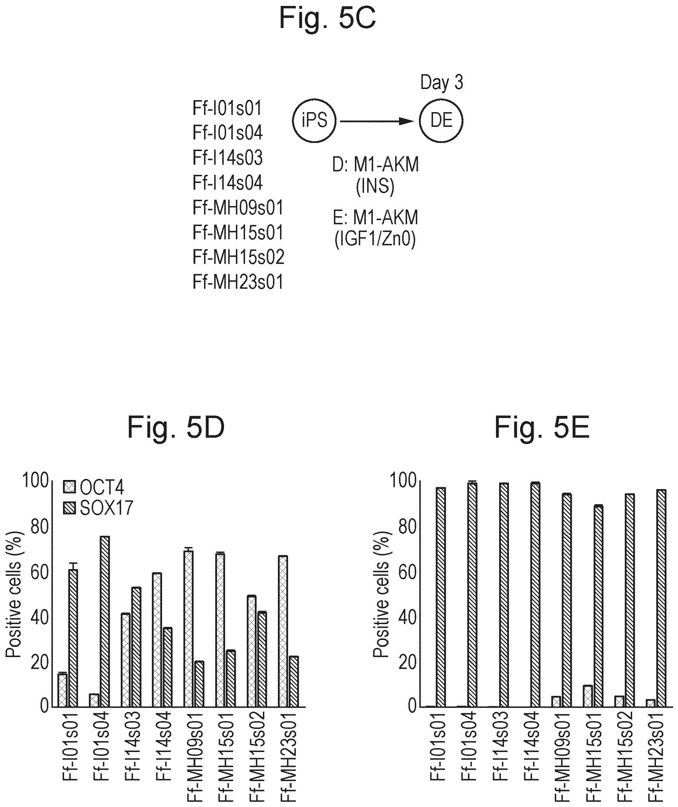

[Figure 5] Deprival of Zn from differentiation media potentiated differentiation into DE cells. A) The concentrations of Zn in the media used in this experiment are shown. B) Primer array analyses were performed to compare among cells cultured in a StemFit AKM (= StemFit AK03N medium without Supplement C). Comparisons are performed for undifferentiated iPSCs cultured in a StmeFit AKM supplemented with insulin; AKM(INS) (red circles) or StmeFit AKM supplemented with IGF1; AKM (IGF1) (blue circles), differentiation day 1 differentiated iPSCs (yellow circles) and differentiation day 3 DE cells (deep yellow). No obvious differences are observed among the undifferentiated cells. C) A schematic drawing of culture conditions, in which 8 hiPSC lines derived from HLA homozygous donors were differentiated into DE under M1-AKM (INS). D) To manipulate Zn concentration, ΔZn AKM medium was used, and IGF1 was used to substitute insulin. Zn0; basal medium deprived of Zn. OCT3/4 and SOX17 positivity are shown. OCT3/4+ cells are reduced when IGF1 was used instead of insulin, and are further reduced at IGF1 and Zn 0. N = 3. (E) Zn deprived condition for differentiating hiPSCs is applicable to 8 hiPSC lines derived from HLA homozygous patients, in which OCT4+ cells are reduced to a minimum level in M1 containing Zn 0 and IGF1. Data are expressed as the mean ± SEM. Differences between groups were analyzed by Student's t-test. Significance is shown as * p < 0.05 or ** p < 0.01.

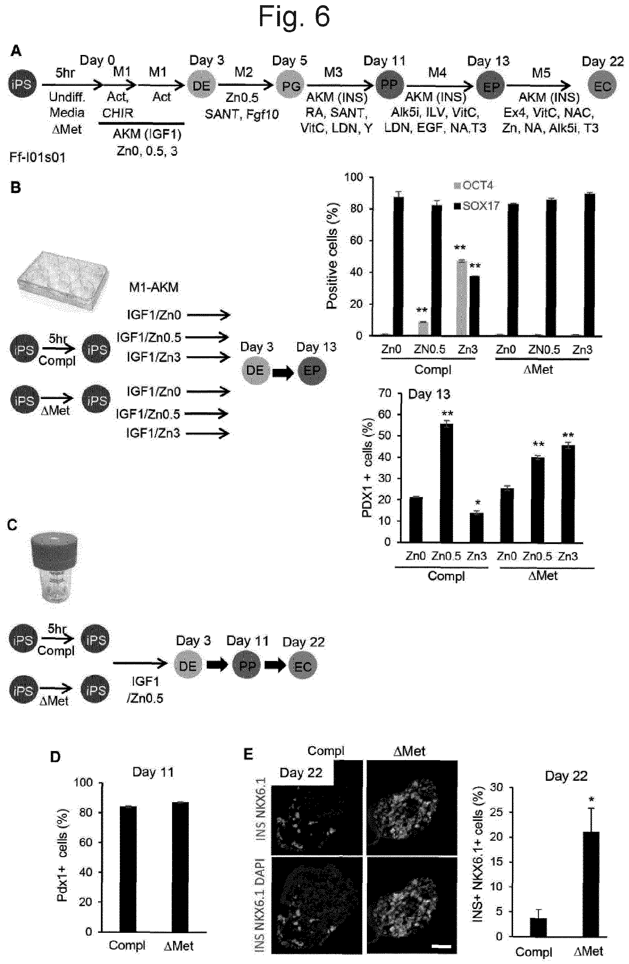

[Figure 6] Deprival of methionine and Zn potentiated differentiation into a pancreatic fate. A) A schematic drawing of the xeno-free culture procedure to obtain insulin-positive EC cells is shown. B) A schematic drawing of the experiment is shown in the left. Zn was added to adjust final concentrations to 0, 0.5 and 3 µM in Medium 1 (M1-IGF1/ Zn0). Methionine pretreatment was done to test if Met deprivation could act with lowered Zn concentration to further reduce OCT3/4+ cells. The result shown in the right revealed that differentiation at higher Zn at 0.5 µM or 3 µM in Medium1 increased OCT3/4+ cells, and Met deprival pretreatment could further reduced the OCT3/4+ cells, and reciprocally increased SOX17+ cells. This was also effective on the generation of PDX1-positive cells on differentiation day 13. Zn at 0.5 µM during M1 phase shows a greatest induction of PDX1-positive cells on differentiation day 13 EP cells. C) A schematic drawing of the xeno-free culture procedure to obtain insulin-positive EC cells from HLA homozygous donor-derived iPSCs. HLA homozygous donor-derived iPSCs were differentiated into endoderm under medium IGF1/Zn 0.5 with (or without methionine deprival, then switched to AKM medium as noted in A from day 3, and continued to differentiate until day 22. D) There was no difference in PDX1+ cells between with or without methionine deprival. E) More INS+ and Nkx6.1+ were yielded in cells treated with methionine deprival at the end of culture (day 22).

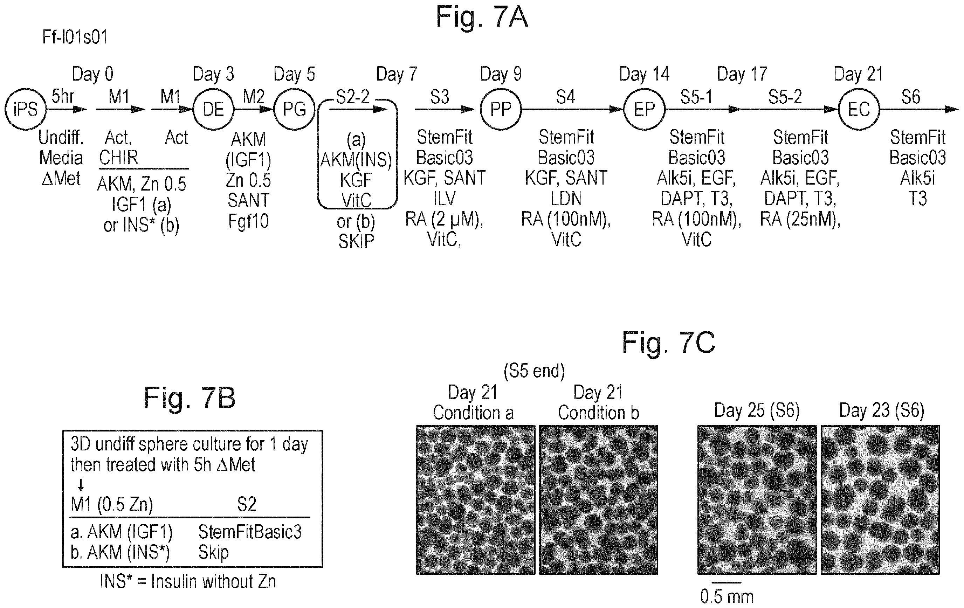

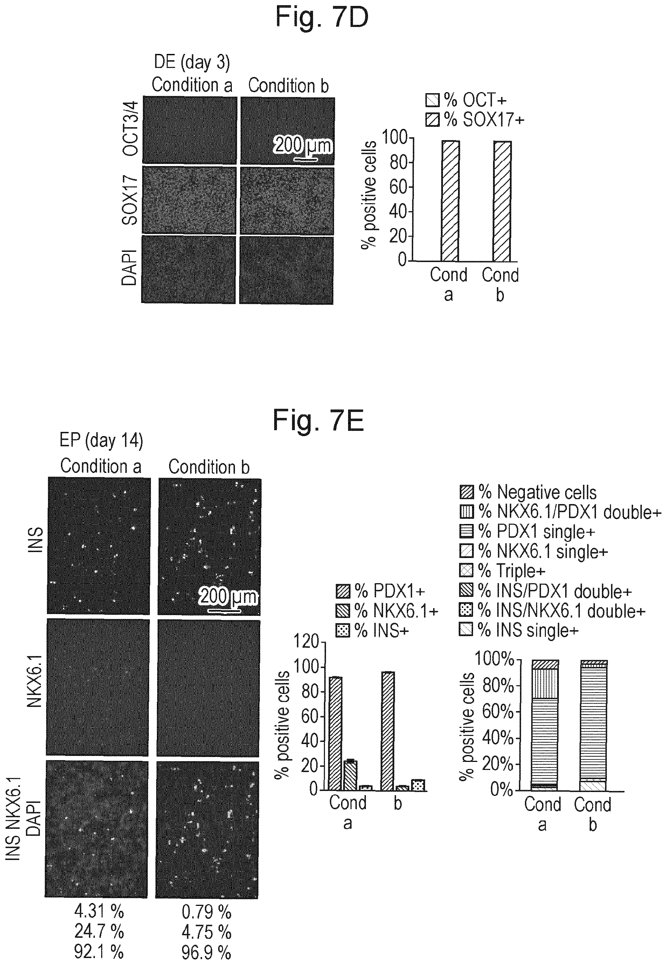

[Figure 7] Methionine deprival and low Zn procedure potentiated differentiation into pancreatic endocrine beta cells. (A) A schematic drawing of the revised differentiation procedure to obtain insulin-positive cells. Met deprival pretreatment and low Zn AKM medium was used during M1 and M2 periods. (B) Conditions tested in the present study. (C) Bright field pictures showing the morphologies of the spheres on the indicated days under conditions a & b. (D) Immunocytochemical analysis on day 3 DE cells of conditions a & b. High SOX17-positive cells are observed in both conditions. (E) Immunocytochemical analysis of PDX1 (blue), NKX6.1 (red) and INS (green) on day 14 EP (Endocrine Progenitor) cells of conditions a & b. Percentages of positive cells are shown. Data are expressed as the mean ± SEM. N = 3. Scale bars, 0.5 mm (C), 200 µm (D, E).

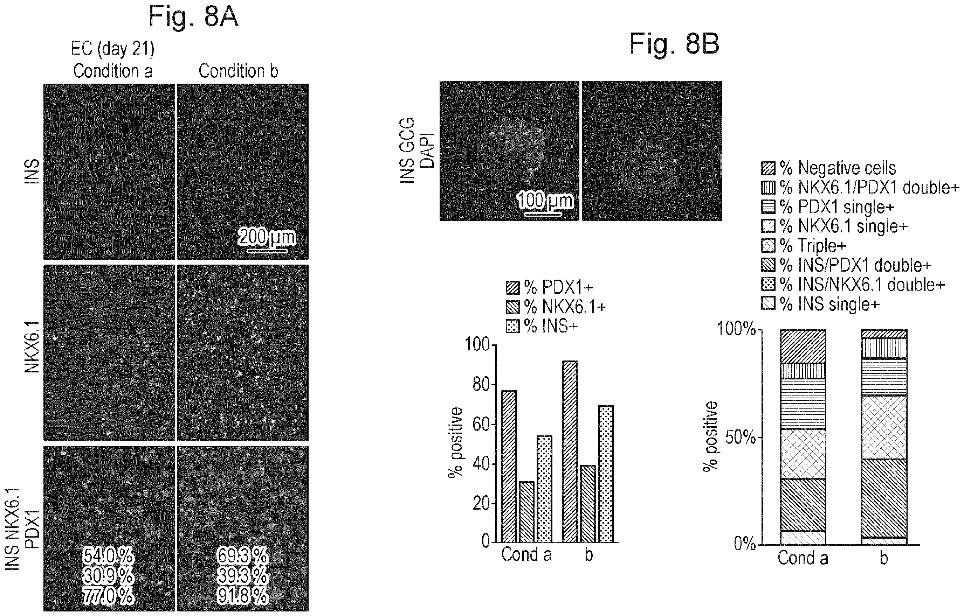

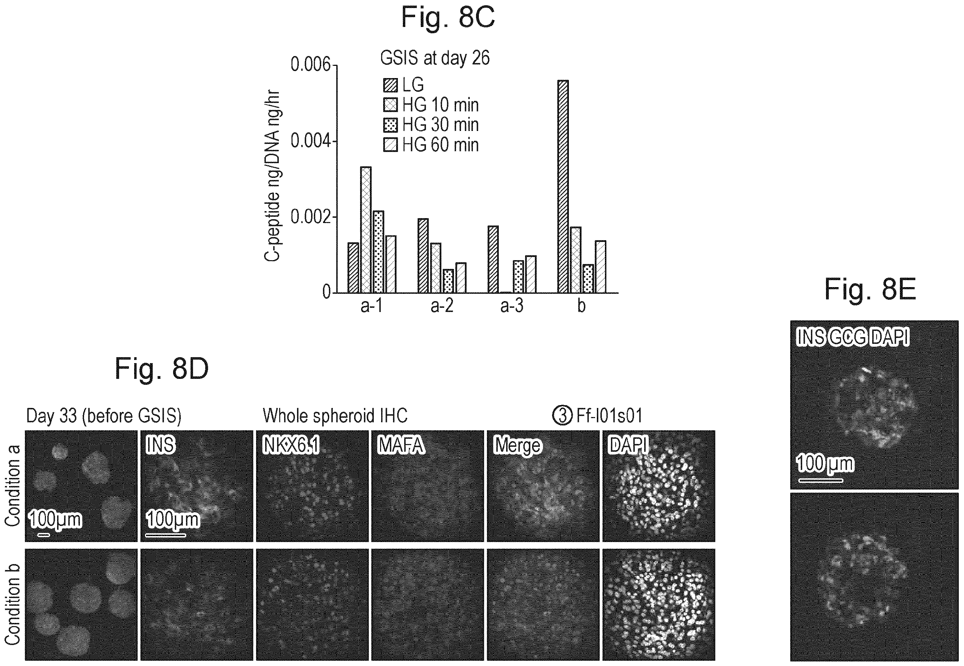

[Figure 8] Methionine deprival and low Zn procedure yielded potentiated differentiation into pancreatic endocrine beta cells. (A) Immunocytochemical analysis of PDX1 (blue), NKX6.1 (red) and INS (green) on day 21 EC (Endocrine cells) of conditions a & b. Percentages of positive cells are shown in graphs. (B) Immunocytochemical analysis of INS (green), glucagon (GCG; red)) and nuclei (DAPI, blue) on day 21 EC (Endocrine cells) of conditions a & b. (C) Glucose-stimulatory insulin secretion activities are assayed on day 26. Normalized C-peptide measurements are shown at each time point (after exposure to Low glucose (LG, 2.5 mM), 10, 30 and 60 min after exposure to high glucose (HG, 20 mM). Cells differentiated under condition a (triplicates) and b are shown. (D) Fluorescence images of immunocytochemical analysis of INS (green), NKX6.1 (red), MAFA (blue) and DAPI (white) are shown. (E) Fluorescence images of immunocytochemical analysis of INS (green), GCG (red) and DAPI (blue) are shown. Data are expressed as the mean ± SEM. N = 3. Scale bars, 200 µm (A), 100 µm (B, D, E) .

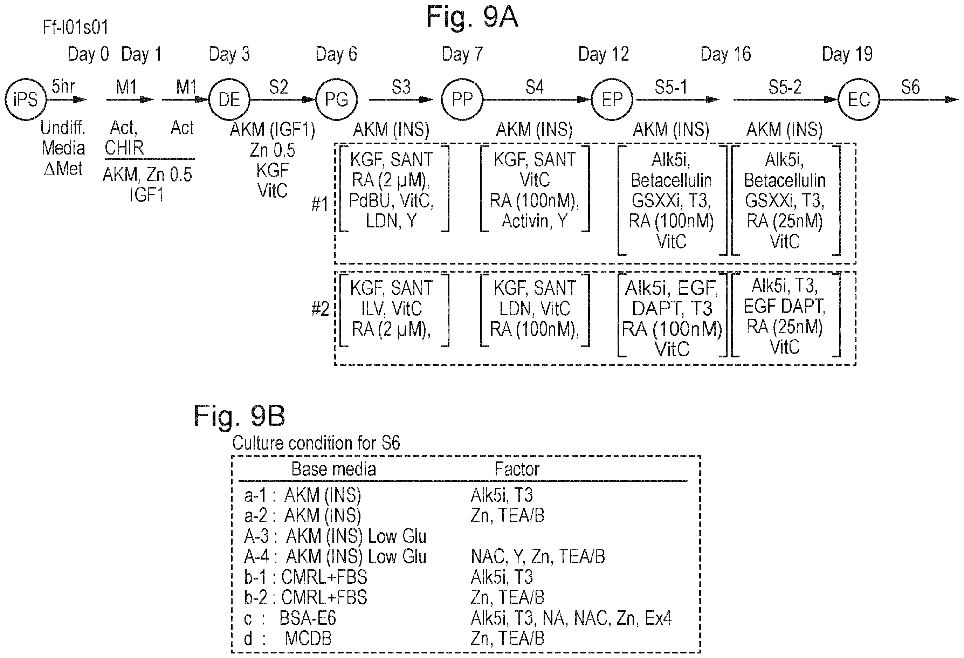

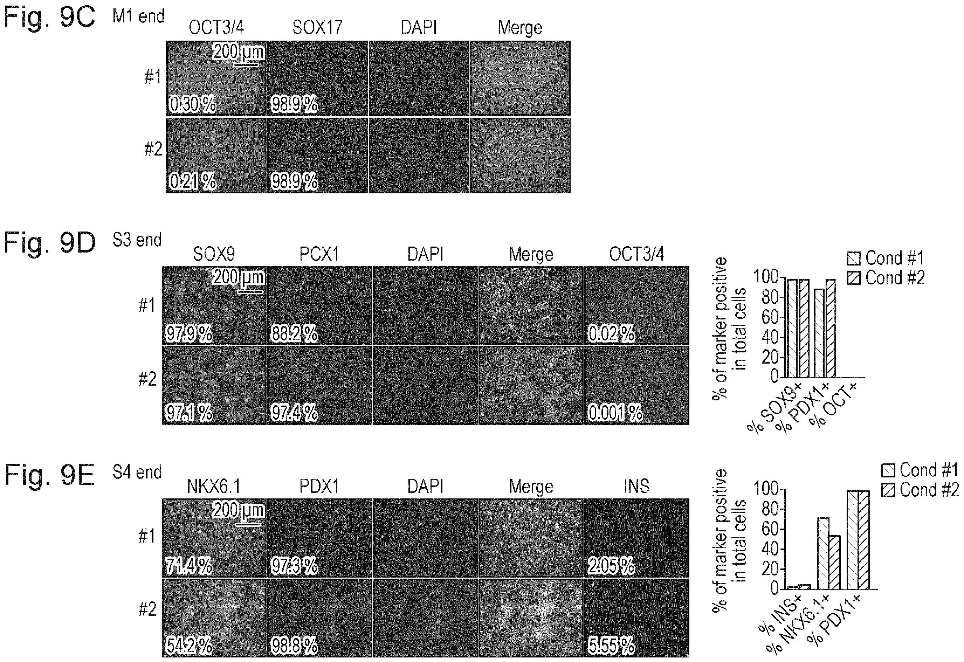

[Figure 9] Methionine deprival and low Zn procedure potentiated differentiation into pancreatic endocrine progenitor cells. (A) A schematic drawing of the revised differentiation procedure to obtain insulin-positive cells. Met deprival pretreatment and low Zn AKM medium was used during M1 and S2 periods. Then from S3 through S5, cells were cultured either in condition #1 or #2, prior to S6 stage. (B) Conditions for S6 stage tested in the present study. (C) Immunocytochemical analysis on day 3 DE cells of conditions #1 & #2. High SOX17+ and low OCT3/4+ cells are observed in both conditions. (D) Immunocytochemical analysis of SOX9 (green), PDX1 (red), OCT3/4 (white) and DAPI (blue) on day 7 PP (pancreatic progenitor) cells of conditions #1 & #2. Percentages of positive cells are shown (in the photo & right graph). (E) Immunocytochemical analysis of PDX1 (red), NKX6.1 (green) and INS (white) and DAPI (blue) on day 12 EP (endocrine progenitor) cells of conditions #1 & #2. Percentages of positive cells are shown (right graph). Data are expressed as the mean ± SEM. N = 3. Scale bars, 200 µm (C, D, E).

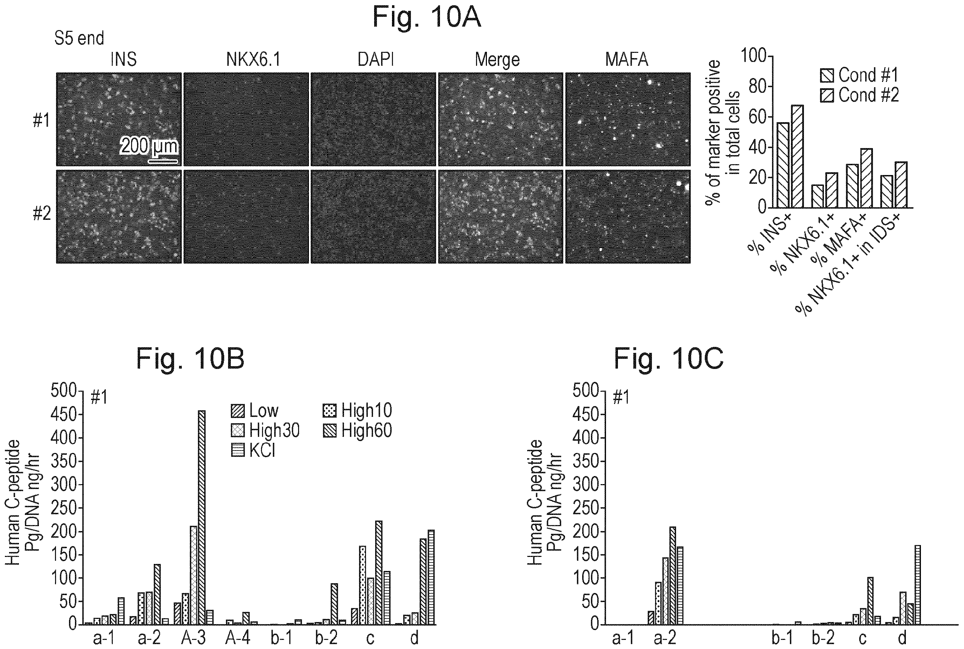

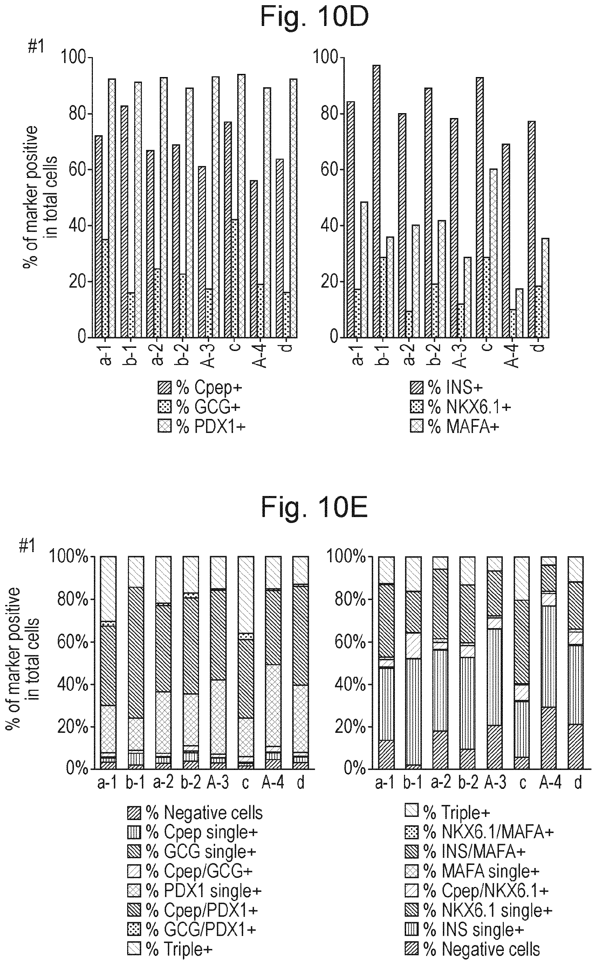

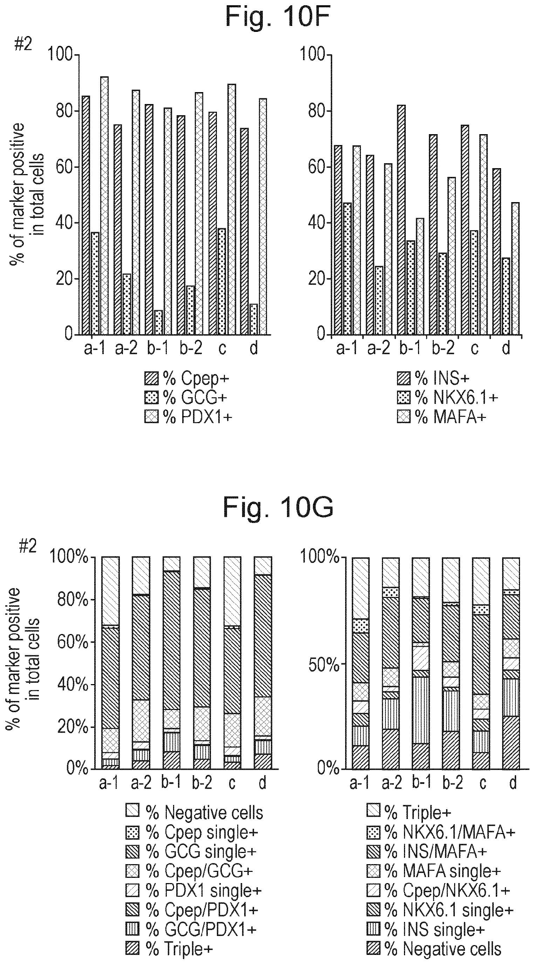

[Figure 10] Methionine deprival and low Zn procedure yielded potentiated differentiation into pancreatic endocrine beta cells. (A) Immunocytochemical analysis of INS (green), NKX6.1 (red), MAFA (white) and DAPI (blue) of conditions #1 & #2 are shown. Percentages of positive cells are shown (right graph). (B) Glucose-stimulatory insulin secretion activities are assayed on day 29, at the end of S6. (C) Normalized C-peptide measurements are shown at each time point (after exposure to low glucose (LG, 2.5 mM), 10, 30 and 60 min after exposure to high glucose (HG, 20 mM). Cells differentiated in different conditions during S6, under condition #1 (B) and #2 (C) are shown. (D-G) Immunocytochemical analyses by triple staining of C-Pep, glucagon (GCG), PDX1, or INS, NKX6.1, MAFA were performed. Percentage of marker positive cells among total cells are shown (D, F). Percentage of marker+ cells among the 3 markers are calculated. Data are expressed as the mean ± SEM. N = 3. Scale bars, 200 µm.

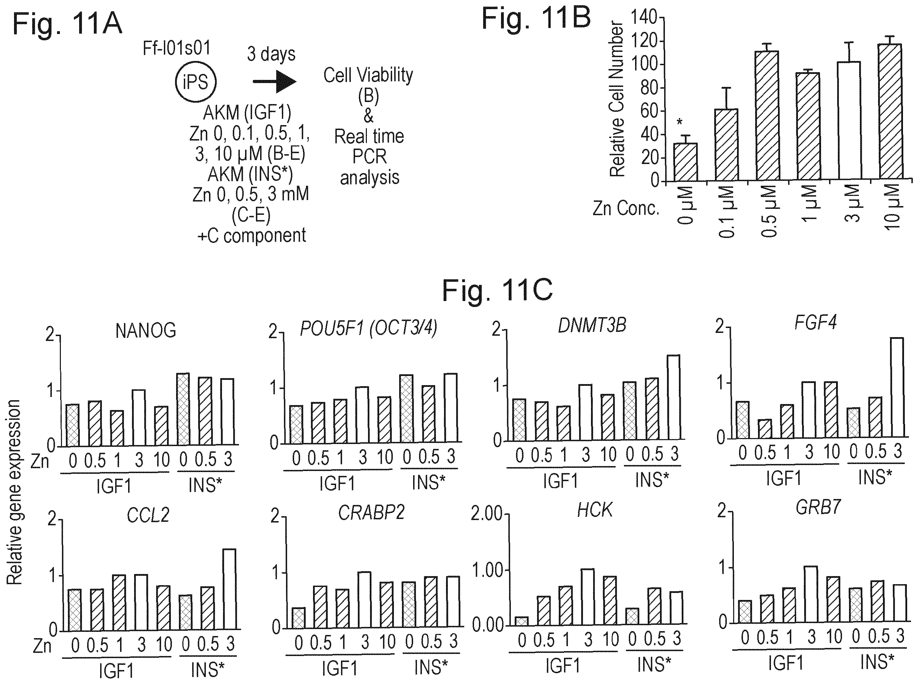

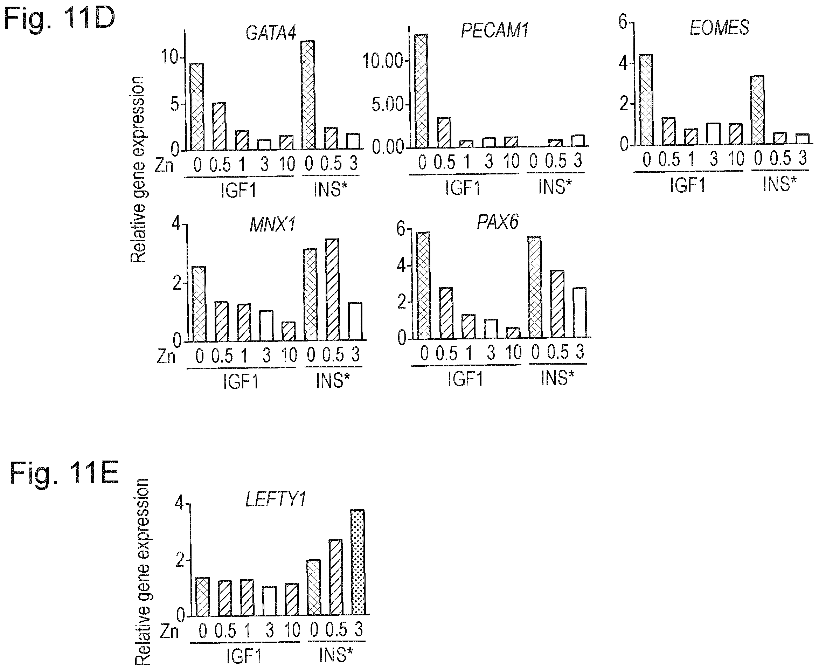

[Figure 11] Effects of low Zn concentrations on undifferentiated iPSCs. Undifferentiated Ef-I01s01 HLA homozygous patient derived iPSCs are cultured for 3 days under graded concentrations of Zn using AKM medium, added with IGF1 or INS* (a mutant INS without Zn), and are assayed for cell viability, and gene expressions. A) A schematic drawing of the experiment. B) Relative cell numbers obtained from cell viability assays are shown. A significant decrease in undifferentiated iPS cell number at Zn 0 µM versus control Zn 3 µM is observed. C) Expressions of pluripotent stem cell markers (NANOG, OCT3/4) or cell growth markers (DNMT3, FGF4, CCL2, CRABP2, HCK, GRB7) or D) Differentiation markers (GATA4, PECAM1, EOMES, MNX1, PAX6) and (E) LEFTY1 were analyzed by real time PCR analysis. White bars, Zn concentration = 3 µM. Black bars, Zn concentration = 0 µM. For B, N = 3. Data are expressed as the mean ± SEM. Differences between groups were analyzed by Student's t-test. Significance is shown as * p < 0.05.

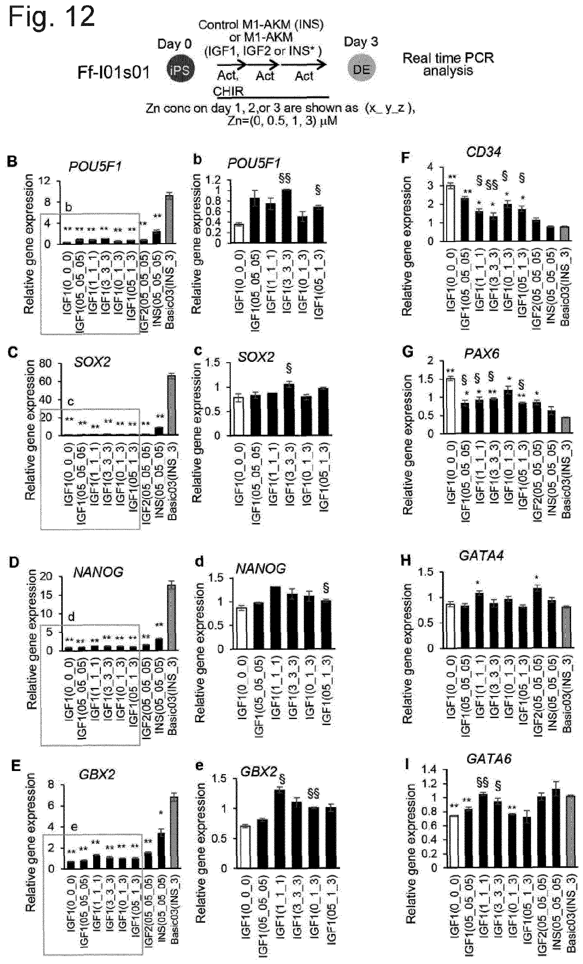

[Figure 12] Potentiated differentiation of Ff-I01s01 HLA iPS cells into endoderm using IGF1, IGF2 and INS* [glulisine (Apidra)] instead of INS and at low Zn condition. A) A schematic drawing of the experiment. Human iPSCs are differentiated to endoderm by culturing for 3 days under control StemFit Basic03 or M1-AKM (IGF1, IGF2, INS*), Zn (0, 0.5, 1, 3) or (INS* is insulin containing no Zn). B-E, b-e) Relative expression levels of POU5F1, SOX2, NANOG or GBX2, or F-I) Relative expression levels of CD34, PAX76, GATA4 or GATA6 are analyzed by real time PCR. N = 3. Data are expressed as the mean ± SEM. Differences between groups were analyzed by Student's t-test. Significance is shown as *p < 0.05 or **p < 0.01; when compared to M1-AKM(INS) or ^{§}p < 0.05, ^{§§}p < 0.01, when compared to M1-AKM [IGF1 (0_0_0)].

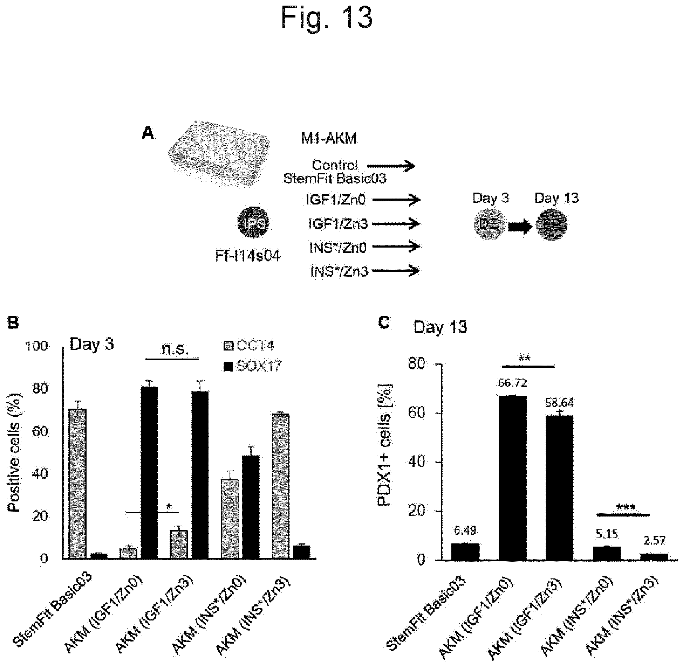

[Figure 13] Ff-I14s04 cells are potentiated to differentiate into endoderm and pancreatic progenitor cells under Zn deprived condition. A) A schematic drawing of the experiment. Human iPSCs are differentiated to endoderm by culturing under control StmeFit Basic03 or M1-AKM (IGF/Zn0 or Zn3) or M1-AKM (INS*/Zn0 or Zn3) (INS* is insulin containing no Zn). B) The ratio of OCT3/4-or SOX17-expressing cells, or C) The ratio of PDX1-expressing cells is evaluated by immunocytochemistry. N = 3. Data are expressed as the mean ± SEM. Differences between groups were analyzed by Student's t-test. Significance is shown as * p < 0.05, ** p < 0.01, or *** p < 0.005. For B), only those that are not significant compared to IGF/Zn3 or INS*/Zn3 are shown.

### Description of Embodiments

Hereinafter, the present invention will be described in detail.

### (A) Method for promoting differentiation of pluripotent stem cells

The method for promoting differentiation of pluripotent stem cells of the present invention comprises a step of culturing pluripotent stem cells in a medium, wherein the medium is a medium comprising an insulin-like growth factor (IGF).

The medium for culturing pluripotent stem cells may be a medium allowing the pluripotent stem cells to differentiate into mesodermal cells or ectodermal cells but preferably is a medium allowing the cells to differentiate into endoderm cells.

The "pluripotent stem cells" refer to cells that have self-renewal ability and can be cultured in vitro and have pluripotency to be capable of differentiating into cells constituting an individual. Specifically, examples thereof can include embryonic stem cells (ES cells), pluripotent stem cells derived from fetal primordial germ cells (GS cells), induced pluripotent stem cells derived from somatic cells (iPS cells), and somatic stem cells. Among them, iPS cells or ES cells are preferably used in the present invention, and human iPS cells and human ES cells are particularly preferable.

The ES cells are preferably ES cells derived from mammals. Examples of the mammals can include mouse, rat, guinea pig, hamster, rabbit, cat, dog, sheep, bovine, horse, goat, monkey, or human, preferably mouse or human, further preferably human.

Generally, the ES cells can be established eventually as a cell line by culturing fertilized eggs in the blastocyst stage together with feeder cells, separating cells derived from inner cell mass proliferated, and further repeating the subculture operation.

Further, the iPS cells refer to cells that have acquired pluripotency, specifically, cells that have acquired pluripotency equivalent to ES cells by introducing several types of transcription factor (pluripotency factor) genes that impart pluripotency to somatic cells (such as fibroblasts). Many factors have been reported as "pluripotency factors", and examples thereof can include, but not specifically limited to, Oct family (such as Oct3/4), Sox family (such as Sox2, Sox1, Sox3, Sox15, and Sox17), Klf family (such as Klf4 and Klf2), Myc family (such as c-Myc, N-Myc, and L-Myc), Nanog, and LIN28. There have been many reports on methods for establishing iPS cells, and they can be referred to (such as Takahashi et al., Cell, 2006, 126:663-676; Okita et al., Nature, 2007, 448:313-317: Wernig et al., Nature, 2007, 448:318-324; Maherali et al., Cell Stem Cell, 2007, 1:55-70; Park et al., Nature, 2007, 451:141-146; Nakagawa et al., Nat Biotechnol 2008, 26:101-106; Wernig et al., Cell Stem Cell, 2008, 10:10-12; Yu et al., Science, 2007, 318:1917-1920; Takahashi et al., Cell, 2007, 131:861-872; Stadtfeld et al., Science, 2008, 322:945-949).

The pluripotent stem cells can be cultured and maintained in a methionine-deprived medium, as described below, but may be cultured and maintained by a method commonly used in this field. The ES cells can be cultured by a conventional method. For example, the cells can be maintained, using mouse fetal fibroblasts (MEF cells) as the feeder cells, in a medium supplemented with a leukemia inhibitory factor, KSR (knockout serum replacement), fetal bovine serum (FBS), a non-essential amino acid, L-glutamine, pyruvic acid, penicillin, streptomycin, or β-mercapto ethanol, such as a DMEM medium. The iPS cells can be cultured also by a conventional method. For example, the cells can be maintained, using MEF cells as the feeder cells, in a medium supplemented with bFGF, KSR (knockout serum replacement), a non-essential amino acid, L-glutamine, penicillin, streptomycin, or β-mercapto ethanol, such as a DMEM/F12 medium.

The medium for culturing pluripotent stem cells contains IGF. Although IGF1 includes IGF1 and IGF2, IGF1 is preferably used in the present invention. The IGF may be derived from any animal but is preferably derived from the same animal as the pluripotent stem cells. For example, in the case of culturing human pluripotent stem cells, it is preferable to use human IGF. The concentration of IGF in the medium is not particularly limited, as long as it can promote the differentiation of pluripotent stem cells, but is preferably 10 to 200 ng/mL, more preferably 50 to 150 ng/mL, further preferably 80 to 120 ng/mL.

The medium for culturing pluripotent stem cells may contain an insulin analogue preparation containing no zinc or an insulin analogue preparation containing a low concentration of zinc, instead of IGF. Here, the "insulin analogue" refers to a peptide in which the amino acid sequence of natural insulin is partially modified, and which has a physiological action similar to that of natural insulin. Further, the "insulin analogue preparation" refers to insulin analogue itself or insulin analogue supplemented with another component. The insulin analogue preparation preferably contains no zinc but may contain a low concentration of zinc. Here, the "low concentration" means that the concentration of zinc in the preparation is lower than that in the insulin (natural insulin) preparation. In the insulin preparation, six molecules of insulin form a crystal structure with diatomic zinc. In the case where the ratio of zinc to the insulin analogue is lower than that in this structure (that is, less than 1/3), the concentration can be a "low concentration". Examples of the insulin analogue preparation containing no zinc can include an insulin glulisine preparation (product name: Apidra). Since the insulin analogue contained in the medium for culturing pluripotent stem cells is considered to exert the same action as insulin, it is considered that the concentration of the insulin analogue in the medium may be the same as the concentration of insulin. The concentration of insulin to be added to the medium for culturing pluripotent stem cells is well known to those skilled in the art, and thus the concentration of the insulin analogue in the medium can be determined accordingly.

Further, the medium for culturing pluripotent stem cells may contain a compound exhibiting an insulin-like action instead of IGF. Many compounds exhibiting an insulin-like action are known, and such known compounds can be used in the present invention. Examples of the compound exhibiting an insulin-like action can include PTP1B (protein tyrosine phosphatase 1B) inhibitors such as compound M (Compound 2 described in Qureshi SA, et al., J Biol Chem. 2000 Nov 24; 275 (47): 36590-5), L-783281 (Zhang B, et al., Science. 1999 May 7; 284 (5416): 974-7), TLK-19781 (Cheng M, et al., J Cell Biochem. 2004 Aug 15; 92 (6): 1234-45 and Lum RT, et al., J Med Chem. 2008 Oct 9; 51 (19): 6173-87), Na₃VO₄ (Green A., Biochem J. 1986 Sep 15; 238 (3): 663-9), and ertiprotafib (International Publication No. WO 1999/061435). Further, the compound described in claim 1 of International Publication No. WO 2017/188082, particularly, compounds 1 to 5 described in Examples can also be used as the compounds exhibiting an insulin-like action.

Since IGF, the insulin analogue preparation, and the compounds exhibiting an insulin-like action are used instead of insulin, the medium for culturing pluripotent stem cells preferably contains no insulin.

The medium for culturing pluripotent stem cells preferably contains zinc at a concentration of 1 µM or less or no zinc. The concentration of zinc needs only to be 1 µM or less and may be 0.9 µM or less, 0.8 µM or less, 0.7 µM or less, 0.6 µM or less, or 0.5 µM or less. Since a small amount of zinc is often contained in the medium, the amount of zinc added to the medium is not necessarily the same as the amount of zinc contained in the medium. Therefore, the medium is treated with a zinc chelating agent (such as Chelex 100) to remove zinc, and then zinc may be added to a predetermined concentration, as shown in Examples described below. Further, the medium is treated with a zinc chelating agent, and then the medium with no zinc added may be used as the "medium containing no zinc". The chelating agent that chelates zinc generally also chelates other metals in addition to zinc (such as cobalt, iron, magnesium, and calcium) and therefore the metals removed are added to the medium after the treatment with the zinc chelating agent.

The medium that allows the pluripotent stem cells to differentiate into endoderm cells preferably contains an activin receptor-like kinase-4,7 activator.

The activin receptor-like kinase (ALK)-4,7 activator is selected from substances that have an activating effect on ALK-4 and/or ALK-7. Examples of the activin receptor-like kinase-4,7 activator to be used include activins, Nodal, and Myostatin, preferably activins. Activins A, B, C, D, and AB are known as activins, and any of the activins can be used. The activin to be used is particularly preferably activin A. Further, the activin to be used can be activins derived from any mammal such as human and mouse, but activins derived from the same animal species as the stem cells used for differentiation are preferably used. For example, in the case where human-derived pluripotent stem cells are used as starting materials, a human-derived activin, particularly, human-derived activin A is preferably used. These activins are commercially available. The concentration of the activin receptor-like kinase (ALK)-4,7 activator in the medium is appropriately set depending on the type to be used but is preferably 3 to 500 ng/mL, more preferably 5 to 200 ng/mL, further preferably 6 to 150 ng/mL, in the case of using human activin A. Activins can cause differentiation of pluripotent stem cells into endoderm in the presence of dimethylsulfoxide even at a low concentration (Ogaki S et al. Sci Rep 5: 17297, 2015) but cannot cause differentiation of pluripotent stem cells into endoderm in the absence of dimethylsulfoxide unless the concentration is increased. Accordingly, in the case where the medium contains no dimethylsulfoxide, the concentration of human activin A is preferably set to a high concentration. Specifically, the concentration is preferably 20 to 500 ng/mL, more preferably 50 to 200 ng/mL.

The medium may contain a GSK3 inhibitor other than the activin receptor-like kinase-4,7 activator. Further, the culture may be divided into two stages, and a medium containing the activin receptor-like kinase-4,7 activator and the GSK3 inhibitor may be used in the first half culture, whereas a medium containing the activin receptor-like kinase-4,7 activator but no GSK3 inhibitor may be used in the latter culture.

The GSK3 inhibitor is selected from the group consisting of substances having GSK3α inhibitory activity, substances having GSK3β inhibitory activity, and substances having both GSK3α inhibitory activity and GSK3β inhibitory activity. Substances having GSK3β inhibitory activity or substances having both GSK3α inhibitory activity and GSK3β inhibitory activity are preferable as the GSK3 inhibitor. Specifically, examples of the GSK3 inhibitor include CHIR98014, CHIR99021, Kenpaullone, AR-AO144-18, TDZD-8, SB216763, BIO, TWS-119, and SB415286. These can be purchased as commercial products. Further, even if they are not available as commercial products, those skilled in the art can prepare them according to known literatures. Further, antisense oligonucleotides and siRNA against the mRNA of GSK3 can also be used as the GSK3 inhibitor. All of these are commercially available or can be synthesized according to known literatures. The GSK3 inhibitor to be used is preferably selected from the group consisting of CHIR99021 (6-[[2-[[4-(2,4-dichlorophenyl)-5-(4-methyl-1H-imidazole-2-yl)-2-pyrimidinyl]amino]ethyl]amino]nicotinonitrile), SB216763 (3-(2,3-dichlorophenyl)-4-(1-methyl-1H-indole-3-yl)-1H-pyrrole-2,5-dione), and SB415286 (3-[(3-chloro-4-hydroxyphenyl)amino]-4-(2-nitrophenyl)-1H-pyrrole-2,5-dione), particularly preferably CHIR99021. The concentration of the GSK3 inhibitor in the medium is appropriately set depending on the type of the GSK3 inhibitor to be used, but the concentration is preferably 1 to 10 µM, more preferably 2 to 5 µM, in the case of using CHIR99021 as the GSK3 inhibitor.

The medium that allows the pluripotent stem cells to differentiate into endoderm cells can be produced based on a known basal medium. Examples of the known basal medium can include BME medium, BGjB medium, CMRL 1066 medium, Glasgow MEM medium, Improved MEM medium, IMDM medium, Medium 199 medium, Eagles MEM medium, αMEM medium, DMEM medium, ham medium, RPMI 1640 medium, Fischer's medium, William's E medium, and a mixed medium thereof, but there is no particular limitation as long as it is a medium that can be used for culturing animal cells.

The medium that allows the pluripotent stem cells to differentiate into endoderm cells may contain a serum replacement. Examples of the serum replacement include albumin, transferrin, fatty acids, collagen precursors, trace elements (such as selenium), B-27 supplement, E5 supplement, E6 supplement, N2 supplement, knockout serum replacement (KSR), 2-mercapto ethanol, 3'-thiol glycerol, or these equivalents. These serum replacements are commercially available. Preferably, examples thereof can include xeno-free B-27 supplement or xeno-free knockout serum replacement (KSR). For example, they can be added to the medium at a concentration of 0.01 to 10 wt%, preferably 0.1 to 2.0 wt%. Further, this medium may contain other additives such as lipids, amino acids (such as non-essential amino acids), vitamins, growth factors, cytokines, antioxidants, 2-mercapto ethanol, pyruvic acid, buffers, inorganic salts, antibiotics (such as penicillin and streptomycin), or antibacterial agents (such as amphotericin B).

The culture in the medium that allows the pluripotent stem cells to differentiate into endoderm cells is generally performed in a CO₂ incubator at a culture temperature suitable for cell culture (generally 30 to 40°C, preferably about 37°C). The culture period is generally 1 day to 5 days, preferably 3 days to 4 days. Further, the culture period may be determined by confirming that the pluripotent stem cells in the culture have differentiated into endoderm cells. The differentiation of the pluripotent stem cells into endoderm cells can be confirmed by evaluating changes in expression of proteins and genes (endoderm markers) that are specifically expressed in endoderm cells. The changes in expression of the endoderm markers can be evaluated, for example, by the method for evaluating protein expression using antigen-antibody reaction or the method for evaluating gene expression using quantitative RT-PCR. Examples of the endoderm markers can include SOX17 and FOXA2.

The pluripotent stem cells may be cultured in an undifferentiated maintenance medium containing no methionine according to a known method (International Publication No. WO 2015/125662) before differentiation into endoderm cells, mesodermal cells, or ectodermal cells. This can further promote the differentiation efficiency of the pluripotent stem cells. Here, "containing no methionine" means to contain 10 µM or less, preferably 5 µM or less, more preferably 1 µM or less, further preferably 0.1 µM or less of methionine, in addition to not containing methionine at all in the medium. The culture time in the medium containing no methionine is not specifically limited but is preferably 3 to 24 hours, more preferably at least 5 to 24 hours, further preferably at least 10 to 24 hours.

The endoderm cells differentiated from the pluripotent stem cells are further differentiated into endoderm-derived cells, for example, pancreatic cells (such as insulin-producing cells), hepatocytes, and intestinal cells. Such differentiation can be performed according to a known method.

The mesodermal cells differentiated from the pluripotent stem cells are further differentiated into mesoderm-derived cells, for example, blood vessel cells, hematopoietic cells, and mesenchymal cells. Such differentiation can be performed according to a known method.

The ectodermal cells differentiated from the pluripotent stem cells are further differentiated into ectoderm-derived cells, for example, nerve cells. Such differentiation can be performed according to a known method.

### (B) First method for inducing differentiation into insulin-producing cells

The first method for inducing differentiation into insulin-producing cells of the present invention is to induce differentiation of pluripotent stem cells into insulin-producing cells, the method comprising steps (1) to (6) below. Generally, steps (1), (2), (3), (4), (5), and (6) are continuously performed in this order, but other steps may be included between the steps.

In step (1), pluripotent stem cells are cultured in a medium containing no methionine. This step can be performed in the same manner as in the culture method in the undifferentiated maintenance medium containing no methionine described above.

In step (2), the cells obtained in step (1) are cultured in a medium containing an activin receptor-like kinase-4,7 activator and a) an insulin-like growth factor, b) an insulin analogue preparation containing no zinc, c) an insulin analogue preparation containing a low concentration of zinc, or d) a compound exerting insulin-like action and containing zinc at a concentration of 1 µM or less or no zinc. This step can be performed in the same manner as in the method for promoting differentiation of pluripotent stem cells of the present invention described above.

In step (3), the cells obtained in step (2) are cultured in a medium containing an FGF and a hedgehog signaling inhibitor.

Examples of the FGF to be used in this step can include FGF1, FGF2 (bFGF), FGF3, FGF4, FGF5, FGF6, FGF7, FGF8, FGF9, FGF10, FGF11, FGF12, FGF13, FGF14, FGF15, FGF16, FGF17, FGF18, FGF19, FGF20, FGF21, FGF22, and FGF23, preferably FGF2 (bFGF), FGF5, FGF7, and FGF10, further preferably FGF10. These may be natural or recombinant proteins.

The hedgehog signaling inhibitor to be used in this step is not particularly limited, as long as it is a substance having inhibitory activity on hedgehog signaling, and may be a naturally occurring substance or a chemically synthesized substance. Preferable examples of the hedgehog signaling inhibitor include cyclopamine, KAAD-cyclopamine (28-[2-[[6-[(3-phenylpropanoyl)amino]hexanoyl]amino]ethyl]-17β, 23β-epoxyveratraman-3-one), KAAD-cyclopamine analogues, jervine(17,23β-epoxy-3β-hydroxyveratraman-11-one), jervine analogues, SANT-1, and hedgehog pathway blocking antibodies, particularly preferably SANT-1.

A medium obtained by adding an FGF and a hedgehog signaling inhibitor to a basal medium can be used as the medium of step (3). The concentration of the FGF in the medium is appropriately set depending on the type to be used but is preferably 10 to 100 ng/mL, more preferably 30 to 80 ng/mL, in the case of using FGF10. The concentration of the hedgehog signaling inhibitor in the medium is appropriately set depending on the type to be used but is preferably 0.1 to 0.5 µM, more preferably 0.2 to 0.3 µM, in the case of using SANT-1. The basal medium to be used may be the same as the medium used in the method for promoting differentiation of pluripotent stem cells of the present invention. Further, the medium of step (3) may contain a serum replacement and other additives like the medium used in the method for promoting differentiation of pluripotent stem cells of the present invention.

The medium of step (3) may contain zinc. The concentration of zinc in the medium is preferably 0.1 to 1 µM, more preferably 0.3 to 0.8 µM.

The culture in step (3) is generally performed in a CO₂ incubator at a culture temperature suitable for cell culture (generally 30 to 40°C, preferably about 37°C). The culture period is generally 1 to 5 days, preferably 1 to 3 days.

In step (4), the cells obtained in step (3) are cultured in a medium containing a retinoic acid receptor agonist, a hedgehog signaling inhibitor, and a BMP signaling inhibitor.

The retinoic acid receptor (RAR) agonist to be used in this step may be a naturally occurring retinoid, a chemically synthesized retinoid, a retinoic acid receptor agonist compound having no retinoid skeleton, or a natural product having retinoic acid receptor agonist activity. Examples of the natural retinoid having an activity as a RAR agonist can include retinoic acids (including isomers). Examples of the retinoic acids can include, but not limited to, all-trans isomer (tretinoin) and 9-cis-retinoic acid (9-cis RA). Further, chemically synthesized retinoids are known in this technical field. Examples of the retinoic acid receptor agonist compound having no retinoid skeleton include Am80, AM580, TTNPB, and AC55649. Examples of the natural product having retinoic acid receptor agonist activity include honokiol and magnolol. The RAR agonist to be used in this step is preferably a retinoic acid, AM580 (4-[[5,6,7,8-tetrahydro-5,5,8,8-tetramethyl-2-naphthalenyl]carboxyamide]benzoic acid), TTNPB (4-[[E]-2-[5,6,7,8-tetrahydro-5,5,8,8-tetramethyl-2-naphthalenyl]-1-propenyl]benzoic acid), or AC55649 (4'-octyl-[1,1'-biphenyl]-4-carboxylic acid), further preferably a retinoic acid.

Examples of the hedgehog signaling inhibitor to be used in this step include those described in step (3), particularly preferably SANT-1. The hedgehog signaling inhibitor to be used in this step may be the same as or different from that used in step (3).

The BMP signaling inhibitor to be used in this step refers to a compound having inhibitory activity on BMP signaling through binding between a BMP and a BMP receptor (type I or type II), and the inhibitor includes proteinaceous inhibitors and low-molecular weight inhibitors. Examples of the proteinaceous inhibitors include natural inhibitors, NOGGIN, CHORDIN, FOLLISTATIN, CERBERUS, and GREMLIN. NOGGIN is known to inhibit BMP signaling by inhibiting binding of BMP4 to the BMP receptor. Further, examples of the low-molecular weight inhibitors can include compounds that inhibit BMP2, BMP4, BMP6, or BMP7 having the ability to activate a transcription factor SMAD1, SMAD5, or SMAD8. Examples thereof can include DORSOMORPHIN (6-[4-(2-piperidine-1-ylethoxy)phenyl]-3-pyridine-4-ylpyrazolo[1,5-a]pyrimidine) and derivatives thereof. Besides this, examples of the BMP signaling inhibitor can include LDN-193189 (4-(6-(4-piperazine-1-yl)phenyl)pyrazolo[1,5-a]pyridine-3-yl)quinoline) and derivatives thereof. These compounds are commercially available, but in the case of being unavailable as commercial products, they can be prepared according to known literatures. Among them, the BMP signaling inhibitor to be used in this step is particularly preferably LDN-193189.

A medium obtained by adding a retinoic acid receptor agonist, a hedgehog signaling inhibitor, and a BMP signaling inhibitor to a basal medium can be used as the medium of step (4). The concentration of the retinoic acid receptor agonist in the medium is appropriately set depending on the type to be used but is preferably 0.5 to 5 µM, more preferably 1 to 3 µM, in the case of using a retinoic acid. The concentration of the hedgehog signaling inhibitor in the medium is appropriately set depending on the type to be used but is preferably 0.1 to 0.5 µM, more preferably 0.2 to 0.3 µM, in the case of using SANT-1. The concentration of the BMP signaling inhibitor in the medium is appropriately set depending on the type to be used but is preferably 0.01 to 0.5 µM, more preferably 0.05 to 0.3 µM, in the case of using LDN193189. The basal medium to be used may be the same as the medium used in the method for promoting differentiation of pluripotent stem cells of the present invention. Further, the medium of step (4) may contain a serum replacement and other additives like the medium used in the method for promoting differentiation of pluripotent stem cells of the present invention.

The medium of step (4) may contain vitamin C and/or Y27632. The concentration of vitamin C in the medium is preferably 10 to 100 µg/mL, more preferably 20 to 70 µg/mL. The concentration of Y27632 in the medium is preferably 1 to 50 µM, more preferably 5 to 30 µM.

The culture in step (4) is generally performed in a CO₂ incubator at a culture temperature suitable for cell culture (generally 30 to 40°C, preferably about 37°C). The culture period is generally 3 to 10 days, preferably 5 to 7 days.

In step (5), the cells obtained in step (4) are cultured in a medium containing a TGF-βI type activin receptor-like kinase-4,5,7 inhibitor, a protein kinase C activator, and a BMP signaling inhibitor.

The TGF-βI type activin receptor-like kinase-4,5,7 inhibitor to be used in this step is selected from compounds having inhibitory activity on at least one ALK selected from the group consisting of TGF-βI type activin receptor-like kinase (ALK)-4, ALK-5, and ALK-7. Examples of the ALK-4, -5, or -7 inhibitor to be used in this step include SB-431542, SB-505124, SB-525334, A-83-01, GW6604, LY580276, TGF-βI type receptor kinase inhibitor II, TGFβRI kinase inhibitor VIII, and SD-208. These are commercially available, but in the case of being unavailable as commercial products, they can be prepared according to known literatures. Further, antisense oligonucleotides and siRNA against mRNA of ALK-4, -5, or -7 can also be used as the ALK-4, -5, or -7 inhibitor. The ALK-4, -5, or -7 inhibitor to be used in this step is preferably SB-431542 (4-[4-(1,3-benzodioxole-5-yl)-5-(2-pyridinyl)-1H-imidazole-2-yl]-benzamide or hydrates thereof), A-83-01 (3-[6-methyl-2-pyridinyl]-N-phenyl-4-[4-quinolinyl]-1H-pyrazol-1-carbothioamide), TGF-βI type receptor kinase inhibitor II (2-[3-[6-methyl pyridine-2-yl]-1H-pyrazol-4-yl]-1, 5-naphthyridine), or TGFβRI kinase inhibitor VIII (6-[2-tert-butyl-5-[6-methylpyridine-2-yl]-1H-imidazol-4-yl]-quinoxaline), further preferably TGF-βI type receptor kinase inhibitor II.

The protein kinase C activator to be used in this step is not specifically limited, as long as it activates protein kinase C signaling and has an activity to direct endoderm cells to pancreatic specialization. Examples thereof can include indolactam V, (2S,5S)-(E,E)-8-(5-(4-(trifluoromethyl)phenyl)-2,4-pentadienoylamino)benzolactam, phorbol-12-myristate-13-acetate, and phorbol-12,13-dibutyrate (PdBU), preferably indolactam V or phorbol-12,13-dibutyrate.

Examples of the BMP signaling inhibitor to be used in this step include step those described in (4), particularly preferably LDN193189. The BMP signaling inhibitor to be used in this step may be the same as or different from that used in step (4).

A medium obtained by adding a TGF-βI type activin receptor-like kinase-4,5,7 inhibitor, a protein kinase C activator, and a BMP signaling inhibitor to a basal medium can be used as the medium of step (5). The concentration of the TGF-βI type activin receptor-like kinase-4,5,7 inhibitor in the medium is appropriately set depending on the type to be used but is preferably 1 to 10 µM, more preferably 3 to 8 µM, in the case of using TGF-βI type receptor kinase inhibitor II. The concentration of the protein kinase C activator in the medium is appropriately set depending on the type to be used but is preferably 0.1 to 0.5 µM, more preferably 0.2 to 0.4 µM, in the case of using indolactam V. The concentration of the BMP signaling inhibitor in the medium is appropriately set depending on the type to be used but is preferably 0.01 to 0.5 µM, more preferably 0.05 to 0.3 µM, in the case of using LDN193189. The basal medium to be used may be the same as the medium used in the method for promoting differentiation of pluripotent stem cells of the present invention.
Further, the medium of step (5) may contain a serum replacement and other additives like the medium used in the method for promoting differentiation of pluripotent stem cells of the present invention.

The medium of step (5) may contain at least one selected from the group consisting of vitamin C, an EGF (epidermal growth factor) receptor agonist, nicotinamide, and T3.

Examples of the EGF receptor agonist to be used in this step can include an EGF, TGF-α, HB-EGF, amphiregulin, betacellulin, and epiregulin. Among these, an EGF or betacellulin is preferable.

The concentration of vitamin C in the medium is preferably 10 to 100 µg/mL, more preferably 20 to 70 µg/mL. The concentration of the EGF receptor agonist in the medium is appropriately set depending on the type to be used but is preferably 10 to 500 ng/mL, more preferably 30 to 300 ng/mL, in the case of using an EGF. The concentration of nicotinamide in the medium is preferably 1 to 50 mM, more preferably 5 to 30 mM. The concentration of T3 in the medium is preferably 0.1 to 5 µM, more preferably 0.5 to 3 µM.

The culture in step (5) is generally performed in a CO₂ incubator at a culture temperature suitable for cell culture (generally 30 to 40°C, preferably about 37°C). The culture period is generally 1 to 5 days, preferably 1 to 3 days.

In step (6), the cells obtained in step (5) are cultured in a medium containing nicotinamide.

A medium obtained by adding nicotinamide to a basal medium can be used as the medium of step (6). The concentration of nicotinamide in the medium is preferably 1 to 50 mM, more preferably 5 to 30 mM. The basal medium to be used may be the same as the medium used in the method for promoting differentiation of pluripotent stem cells of the present invention. Further, the medium of step (6) may contain a serum replacement and other additives like the medium used in the method for promoting differentiation of pluripotent stem cells of the present invention.

The medium of step (6) may contain at least one selected from the group consisting of a GLP-1 receptor agonist, vitamin C, N-acetyl-L-cysteine, zinc, a TGF-βI type activin receptor-like kinase-4,5,7 inhibitor, and T3.

The GLP-1 receptor agonist to be used in this step is a substance having an activity as an agonist for a GLP-1 (glucagon-like peptide-1) receptor. Examples of the GLP-1 receptor agonist can include GLP-1, a GLP-1MR agent, NN-2211, AC-2993 (exendin-4), BIM-51077, Aib(8,35)hGLP-1(7,37)NH₂, and CJC-1131, particularly preferably exendin-4. These substances are commercially available, but in the case of being unavailable as commercial products, they can be prepared according to known literatures. Besides, many GLP-1 receptor agonists are commercially available, and they can also be used in this step.

Examples of the TGF-βI type activin receptor-like kinase-4,5,7 inhibitor to be used in this step include those described in step (5), particularly preferably TGF-βI type receptor kinase inhibitor II. The TGF-βI type activin receptor-like kinase-4,5,7 inhibitor to be used in this step may be the same as or different from that used in step (5).

The concentration of the GLP-1 receptor agonist in the medium is appropriately set depending on the type to be used but is preferably 10 to 100 ng/mL, more preferably 30 to 80 ng/mL, in the case of using exendin-4. The concentration of vitamin C in the medium is preferably 10 to 100 µg/mL, more preferably 20 to 70 µg/mL. The concentration of N-acetyl-L-cysteine in the medium is preferably 0.1 to 5 mM, more preferably 0.5 to 3 mM. The concentration of the TGF-βI type activin receptor-like kinase-4,5,7 inhibitor in the medium is appropriately set depending on the type to be used but is preferably 1 to 50 µM, more preferably 5 to 30 µM, in the case of using TGF-βI type receptor kinase inhibitor II. The concentration of zinc in the medium is preferably 1 to 50 µM, more preferably 5 to 30 µM. The concentration of T3 in the medium is preferably 0.1 to 5 µM, more preferably 0.5 to 3 µM.

The culture in step (6) is generally performed in a CO₂ incubator at a culture temperature suitable for cell culture (generally 30 to 40°C, preferably about 37°C). The culture period is generally 5 to 15 days, preferably 7 to 12 days.

The medium to be used in steps (1) to (6) is preferably a xeno-free medium.

### (C) Second method for inducing differentiation into insulin-producing cells

The second method for inducing differentiation into insulin-producing cells of the present invention is to induce differentiation of pluripotent stem cells into insulin-producing cells, the method comprising steps (1) to (9), or (1) to (3) and (5) to (9). Generally, steps (1), (2), (3), (4), (5), (6), (7), (8), and (9) or steps (1), (2), (3), (5), (6), (7), (8), and (9) are continuously performed in this order, but other steps may be included between the steps.

In step (1), pluripotent stem cells are cultured in a medium containing no methionine. This step can be performed in the same manner as step (1) in the first method for inducing differentiation into insulin-producing cells.

In step (2), the cells obtained in step (1) are cultured in a medium containing an activin receptor-like kinase-4,7 activator and a) an insulin-like growth factor, b) an insulin analogue preparation containing no zinc, c) an insulin analogue preparation containing a low concentration of zinc, or d) a compound exerting insulin-like action and containing zinc at a concentration of 1 µM or less or no zinc. This step can be performed in the same manner as step (2) in the first method for inducing differentiation into insulin-producing cells.

In step (3), the cells obtained in step (2) are cultured in a medium containing an FGF and a hedgehog signaling inhibitor. This step can be performed in the same manner as step (3) in the first method for inducing differentiation into insulin-producing cells.

In step (4), the cells obtained in step (3) are cultured in a medium containing a KGF (keratinocyte growth factor). This step may be omitted. In the case where the cells obtained in step (1) are cultured in a medium containing an insulin-like growth factor in step (2), it is preferable not to omit this step. Meanwhile, in the case where the cells obtained in step (1) are cultured in a medium containing an insulin analogue preparation with no zinc in step (2), this step may be omitted.

A medium obtained by adding a KGF to a basal medium can be used as the medium of step (4). The concentration of the KGF in the medium is preferably 10 to 100 ng/mL, more preferably 30 to 80 ng/mL. The basal medium to be used may be the same as the medium used in the method for promoting differentiation of pluripotent stem cells of the present invention. Further, the medium of step (4) may contain a serum replacement and other additives like the medium used in the method for promoting differentiation of pluripotent stem cells of the present invention.

The medium of step (4) may contain vitamin C. The concentration of vitamin C in the medium is preferably 10 to 100 µg/mL, more preferably 20 to 70 µg/mL.

The culture in step (4) is generally performed in a CO₂ incubator at a culture temperature suitable for cell culture (generally 30 to 40°C, preferably about 37°C). The culture period is generally 1 to 5 days, preferably 1 to 3 days.

In step (5), the cells obtained in step (3) or step (4) are cultured in a medium containing a KGF, a hedgehog signaling inhibitor, a protein kinase C activator, and a retinoic acid receptor agonist.

Examples of the hedgehog signaling inhibitor to be used in this step include those described in the first method for inducing differentiation into insulin-producing cells, particularly preferably SANT-1.

Examples of the protein kinase C activator to be used in this step include those described in the first method for inducing differentiation into insulin-producing cells, particularly preferably indolactam V.

Examples of the retinoic acid receptor agonist to be used in this step include those described in the first method for inducing differentiation into insulin-producing cells, particularly preferably a retinoic acid.

A medium obtained by adding a KGF, a hedgehog signaling inhibitor, a protein kinase C activator, and a retinoic acid receptor agonist to a basal medium can be used as the medium of step (5). The concentration of the KGF in the medium is preferably 10 to 100 ng/mL, more preferably 30 to 80 ng/mL. The concentration of the hedgehog signaling inhibitor in the medium is appropriately set depending on the type to be used but is preferably 0.1 to 0.5 µM, more preferably 0.2 to 0.3 µM, in the case of using SANT-1. The concentration of the protein kinase C activator in the medium is appropriately set depending on the type to be used but is preferably 10 to 100 nM, more preferably 30 to 80 nM, in the case of using indolactam V. The concentration of the retinoic acid receptor agonist in the medium is appropriately set depending on the type to be used but is preferably 0.5 to 5 µM, more preferably 1 to 3 µM, in the case of using a retinoic acid. The basal medium to be used may be the same as the medium used in the method for promoting differentiation of pluripotent stem cells of the present invention. Further, the medium of step (5) may contain a serum replacement and other additives like the medium used in the method for promoting differentiation of pluripotent stem cells of the present invention.

The medium of step (5) may contain vitamin C. The concentration of vitamin C in the medium is preferably 10 to 100 µg/mL, more preferably 20 to 70 µg/mL.

The culture in step (5) is generally performed in a CO₂ incubator at a culture temperature suitable for cell culture (generally 30 to 40°C, preferably about 37°C). The culture period is generally 1 to 5 days, preferably 1 to 3 days.

In step (6), the cells are cultured in a medium containing a KGF, a hedgehog signaling inhibitor, a retinoic acid receptor agonist, and a BMP signaling inhibitor.

Examples of the hedgehog signaling inhibitor to be used in this step include those described in the first method for inducing differentiation into insulin-producing cells, particularly preferably SANT-1. The hedgehog signaling inhibitor is used also in step (5), and the hedgehog signaling inhibitor to be used in this step may be the same as or different from that used in step (5).

Examples of the retinoic acid receptor agonist to be used in this step include those described in the first method for inducing differentiation into insulin-producing cells, particularly preferably a retinoic acid. The retinoic acid receptor agonist is used also in step (5), and the retinoic acid receptor agonist to be used in this step may be the same as or different from that used in step (5).

Examples of the BMP signaling inhibitor to be used in this step include those described in the first method for inducing differentiation into insulin-producing cells, particularly preferably LDN193189.

A medium obtained by adding a KGF, a hedgehog signaling inhibitor, a retinoic acid receptor agonist, and a BMP signaling inhibitor to a basal medium can be used as the medium of step (6). The concentration of the KGF in the medium is preferably 10 to 100 ng/mL, more preferably 30 to 80 ng/mL. The concentration of the hedgehog signaling inhibitor in the medium is appropriately set depending on the type to be used but is preferably 0.1 to 0.5 µM, more preferably 0.2 to 0.3 µM, in the case of using SANT-1. The concentration of the retinoic acid receptor agonist in the medium is appropriately set depending on the type to be used but is preferably 10 to 500 nM, more preferably 50 to 300 nM, in the case of using a retinoic acid. The concentration of the BMP signaling inhibitor in the medium is appropriately set depending on the type to be used but is preferably 10 to 500 nM, more preferably 50 to 300 nM, in the case of using LDN193189. The basal medium to be used may be the same as the medium used in the method for promoting differentiation of pluripotent stem cells of the present invention. Further, the medium of step (6) may contain a serum replacement and other additives like the medium used in the method for promoting differentiation of pluripotent stem cells of the present invention.

The medium of step (6) may contain vitamin C. The concentration of vitamin C in the medium is preferably 10 to 100 µg/mL, more preferably 30 to 80 µg/mL.

The culture in step (6) is generally performed in a CO₂ incubator at a culture temperature suitable for cell culture (generally 30 to 40°C, preferably about 37°C). The culture period is generally 3 to 7 days, preferably 4 to 6 days.

In step (7), the cells obtained in step (6) are cultured in a medium containing a TGF-βI type activin receptor-like kinase-4,5,7 inhibitor, an EGF receptor agonist, a γ-secretase inhibitor, and a retinoic acid receptor agonist.

Examples of the TGF-βI type activin receptor-like kinase-4,5,7 inhibitor to be used in this step include those described in the first method for inducing differentiation into insulin-producing cells, particularly preferably TGF-βI type receptor-like kinase inhibitor II (ALK5i).

Examples of the EGF receptor agonist to be used in this step include those described in the first method for inducing differentiation into insulin-producing cells, particularly preferably an EGF.

Examples of the γ-secretase inhibitor to be used in this step can include aryl sulfonamide, dibenzazepine, benzodiazepine, DAPT, L-685458, MK0752, or γ-secretase inhibitor XXI (GSXXi). These can be purchased as commercial products. Further, even if they are not available as commercial products, those skilled in the art can prepare them according to known literatures. Suitable examples of the γ-secretase inhibitor can include DAPT or γ-secretase inhibitor XXI.

Examples of the retinoic acid receptor agonist to be used in this step include those described in the first method for inducing differentiation into insulin-producing cells, particularly preferably a retinoic acid. The retinoic acid receptor agonist is used also in steps (5) and (6), and the hedgehog signaling inhibitor to be used in this step may be the same as or different from that used in steps (5) and (6).

A medium obtained by adding a TGF-βI type activin receptor-like kinase-4,5,7 inhibitor, an EGF receptor agonist, a γ-secretase inhibitor, and a retinoic acid receptor agonist to a basal medium can be used as the medium of step (7). The concentration of the TGF-βI type activin receptor-like kinase-4,5,7 inhibitor in the medium is appropriately set depending on the type to be used but is preferably 1 to 50 µM, more preferably 5 to 30 µM, in the case of using TGF-βI type receptor-like kinase inhibitor II. The concentration of the EGF receptor agonist in the medium is appropriately set depending on the type to be used but is preferably 10 to 50 ng/mL, more preferably 30 to 40 ng/mL, in the case of using an EGF. The concentration of the γ-secretase inhibitor in the medium is appropriately set depending on the type to be used but is preferably 1 to 50 µM, more preferably 5 to 30 µM, in the case of using DAPT. The concentration of the retinoic acid receptor agonist in the medium is appropriately set depending on the type to be used but is preferably 10 to 500 nM, more preferably 50 to 300 nM, in the case of using a retinoic acid. The basal medium to be used may be the same as the medium used in the method for promoting differentiation of pluripotent stem cells of the present invention. Further, the medium of step (7) may contain a serum replacement and other additives like the medium used in the method for promoting differentiation of pluripotent stem cells of the present invention.

The medium of step (7) may contain triiodothyronine and/or vitamin C. The concentration of triiodothyronine in the medium is preferably 0.1 to 5 µM, more preferably 0.5 to 3 µM. The concentration of vitamin C in the medium is preferably 10 to 100 µg/mL, more preferably 30 to 80 µg/mL.

The culture in step (7) is generally performed in a CO₂ incubator at a culture temperature suitable for cell culture (generally 30 to 40°C, preferably about 37°C). The culture period is generally 1 to 5 days, preferably 2 to 4 days.

In step (8), the cells obtained in step (7) are cultured in a medium containing a TGF-βI type activin receptor-like kinase-4,5,7 inhibitor, an EGF receptor agonist, a γ-secretase inhibitor, and a retinoic acid receptor agonist. In this step, the concentration of the retinoic acid receptor agonist in the medium is set to a concentration lower than the concentration of the retinoic acid receptor agonist in step (7).

In step (8), the culture can be performed in the same medium as used in step (7) except the concentration of the retinoic acid receptor agonist. The medium of step (8) may contain no vitamin C.

The concentration of the retinoic acid receptor agonist in the medium is not specifically limited, as long as it is lower than the concentration of the retinoic acid receptor agonist in step (7), but is preferably 10 to 50 nM, more preferably 20 to 30 nM, for example, in the case of using a retinoic acid.

The culture in step (8) is generally performed in a CO₂ incubator at a culture temperature suitable for cell culture (generally 30 to 40°C, preferably about 37°C). The culture period is generally 2 to 6 days, preferably 3 to 5 days.

In step (9), the cells obtained in step (8) are cultured in a medium containing a TGF-βI type activin receptor-like kinase-4,5,7 inhibitor.

Examples of the TGF-βI type activin receptor-like kinase-4,5,7 inhibitor to be used in this step include those described in the first method for inducing differentiation into insulin-producing cells, particularly preferably TGF-βI type receptor-like kinase inhibitor II (ALK5i). The TGF-βI type activin receptor-like kinase-4,5,7 inhibitor is used also in steps (7) and (8), and the TGF-βI type activin receptor-like kinase-4,5,7 inhibitor to be used in this step may be the same as or different from that used in steps (7) and (8).

A medium obtained by adding a TGF-βI type activin receptor-like kinase-4,5,7 inhibitor to a basal medium can be used as the medium of step (9). The concentration of the TGF-βI type activin receptor-like kinase-4,5,7 inhibitor in the medium is appropriately set depending on the type to be used but is preferably 1 to 50 µM, more preferably 5 to 30 µM, in the case of using TGF-βI type receptor-like kinase inhibitor II. The basal medium to be used may be the same as the medium used in the method for promoting differentiation of pluripotent stem cells of the present invention. Further, the medium of step (9) may contain a serum replacement and other additives like the medium used in the method for promoting differentiation of pluripotent stem cells of the present invention.

The medium of step (9) may contain triiodothyronine. The concentration of triiodothyronine in the medium is preferably 0.1 to 5 µM, more preferably 0.5 to 3 µM.

The culture in step (9) is generally performed in a CO₂ incubator at a culture temperature suitable for cell culture (generally 30 to 40°C, preferably about 37°C). The culture period is generally 1 to 20 days, preferably 3 to 15 days.

### (D) Method for differentiating pancreatic progenitor cells into pancreatic beta cells

The method for differentiating pancreatic progenitor cells into pancreatic beta cells of the present invention comprises culturing pancreatic progenitor cells in a medium containing a factor selected from the group consisting of a retinoic acid receptor agonist, a hedgehog signaling inhibitor, a KGF, a γ-secretase inhibitor, a BMP signaling inhibitor, a TGF-βI type activin receptor-like kinase-4,5,7 inhibitor, and an EGF receptor agonist, wherein the pancreatic progenitor cells are PDX1 positive or NKX6.1 positive.

Examples of the retinoic acid receptor agonist to be used in the differentiation method of the present invention include those described in the first method for inducing differentiation into insulin-producing cells, particularly preferably a retinoic acid.

Examples of the hedgehog signaling inhibitor to be used in the differentiation method of the present invention include those described in the first method for inducing differentiation into insulin-producing cells, particularly preferably SANT-1.

Examples of the γ-secretase inhibitor to be used in the differentiation method of the present invention include those described in the second method for inducing differentiation into insulin-producing cells, particularly preferably DAPT.

Examples of the BMP signaling inhibitor to be used in the differentiation method of the present invention include those described in the first method for inducing differentiation into insulin-producing cells, particularly preferably LDN193189.

Examples of the TGF-βI type activin receptor-like kinase-4,5,7 inhibitor to be used in the differentiation method of the present invention include those described in the first method for inducing differentiation into insulin-producing cells, particularly preferably TGF-βI type receptor-like kinase inhibitor II (ALK5i).

Examples of the EGF receptor agonist to be used in this step include those described in the first method for inducing differentiation into insulin-producing cells, particularly preferably an EGF.

A medium obtained by adding a factor selected from the group consisting of a retinoic acid receptor agonist, a hedgehog signaling inhibitor, a KGF, a γ-secretase inhibitor, a BMP signaling inhibitor, a TGF-βI type activin receptor-like kinase-4,5,7 inhibitor, and an EGF receptor agonist to a basal medium can be used as the medium for culturing the pancreatic progenitor cells. The concentration of each factor in the medium may be the same as the concentration described in the method for promoting differentiation of pluripotent stem cells, the first method for inducing differentiation into insulin-producing cells, or the second method for inducing differentiation into insulin-producing cells.

### (E) Third method for inducing differentiation into insulin-producing cells

The third method for inducing differentiation into insulin-producing cells of the present invention is to induce differentiation of pluripotent stem cells into insulin-producing cells, the method comprising steps (1) to (7) below. Generally, steps (1), (2), (3), (4), (5), (6), and (7) are continuously performed in this order, but other steps may be included between the steps.

In step (1), pluripotent stem cells are cultured in a medium containing no methionine. This step can be performed in the same manner as in the culture method in the undifferentiated maintenance medium containing no methionine described above.

In step (2), the cells obtained in step (1) are cultured in a medium containing an activin receptor-like kinase-4,7 activator and a) an insulin-like growth factor, b) an insulin analogue preparation containing no zinc, c) an insulin analogue preparation containing a low concentration of zinc, or d) a compound exerting insulin-like action and containing zinc at a concentration of 1 µM or less or no zinc. This step can be performed in the same manner as in the method for promoting differentiation of pluripotent stem cells of the present invention.

In step (3), the cells obtained in step (2) are cultured in a medium containing a KGF and containing zinc at a concentration of 1 µM or less or no zinc.

A medium obtained by adding a KGF to a basal medium can be used as the medium of step (3). The concentration of the KGF in the medium is preferably 10 to 100 ng/mL, more preferably 30 to 80 ng/mL. The basal medium to be used may be the same as the medium used in the method for promoting differentiation of pluripotent stem cells of the present invention. Further, the medium of step (3) may contain a serum replacement and other additives like the medium used in the method for promoting differentiation of pluripotent stem cells of the present invention.

The medium of step (3) contains zinc at a concentration of 1 µM or less or no zinc. The concentration of zinc needs only to be 1 µM or less but may be 0.9 µM or less, 0.8 µM or less, 0.7 µM or less, 0.6 µM or less, or 0.5 µM or less. Since a small amount of zinc is often contained in the medium, the amount of zinc added to the medium may not be the same as the amount of zinc contained in the medium. Therefore, zinc may be added to a predetermined concentration after the medium is treated with a zinc chelating agent (such as Chelex 100) to remove zinc, as shown in Examples described below. Further, after the medium is treated with a zinc chelating agent, the medium with no zinc added may be used as the "medium containing no zinc". Since the chelating agent that chelates zinc generally also chelates metals other than zinc (such as cobalt, iron, magnesium, and calcium), the metals removed are added to the medium after the treatment with the zinc chelating agent.

The medium of step (3) may contain vitamin C. The concentration of vitamin C in the medium is preferably 10 to 100 µg/mL, more preferably 20 to 70 µg/mL.

When a hedgehog signaling inhibitor such as SANT-1 is added to the medium of step (3), the glucose-stimulated insulin secretion of the insulin-producing cells to be obtained may possibly decrease. Therefore, the medium preferably contains no hedgehog signaling inhibitor.

The culture in step (3) is generally performed in a CO₂ incubator at a culture temperature suitable for cell culture (generally 30 to 40°C, preferably about 37°C). The culture period is generally 1 to 5 days, preferably 2 to 4 days.

In step (4), the cells obtained in step (3) are cultured in a medium containing a KGF, a hedgehog signaling inhibitor, and a retinoic acid receptor agonist.

Examples of the hedgehog signaling inhibitor to be used in this step include those described in the first method for inducing differentiation into insulin-producing cells, particularly preferably SANT-1.

Examples of the retinoic acid receptor agonist to be used in this step include those described in the first method for inducing differentiation into insulin-producing cells, particularly preferably a retinoic acid.

A medium obtained by adding a KGF, a hedgehog signaling inhibitor, and a retinoic acid receptor agonist to a basal medium can be used as the medium of step (4). The concentration of the KGF in the medium is preferably 10 to 100 ng/mL, more preferably 30 to 80 ng/mL. The concentration of the hedgehog signaling inhibitor in the medium is appropriately set depending on the type to be used but is preferably 0.1 to 0.5 µM, more preferably 0.2 to 0.3 µM, in the case of using SANT-1. The concentration of the retinoic acid receptor agonist in the medium is appropriately set depending on the type to be used but is preferably 0.5 to 5 µM, more preferably 1 to 3 µM, in the case of using a retinoic acid. The basal medium to be used may be the same as the medium used in the method for promoting differentiation of pluripotent stem cells of the present invention. Further, the medium of step (4) may contain a serum replacement and other additives like the medium used in the method for promoting differentiation of pluripotent stem cells of the present invention.

The medium of step (4) may contain at least one selected from the group consisting of a BMP signaling inhibitor, a protein kinase C activator, vitamin C, and Y27632.

Examples of the BMP signaling inhibitor to be used in this step include those described in the first method for inducing differentiation into insulin-producing cells, particularly preferably LDN193189.

Examples of the protein kinase C activator to be used in this step include those described in the first method for inducing differentiation into insulin-producing cells, particularly preferably indolactam V or phorbol-12,13-dibutyrate.

The concentration of the BMP signaling inhibitor in the medium is appropriately set depending on the type to be used but is preferably 10 to 500 nM, more preferably 50 to 300 nM, in the case of using LDN193189. The concentration of the protein kinase C activator in the medium is appropriately set depending on the type to be used but is preferably 10 to 500 nM, more preferably 30 to 200 nM, in the case of using indolactam V, and is preferably 100 to 1000 nM, more preferably 300 to 800 nM, in the case of using phorbol-12,13-dibutyrate. The concentration of vitamin C in the medium is preferably 10 to 100 µg/mL, more preferably 20 to 70 µg/mL. The concentration of Y27632 in the medium is preferably 1 to 50 µM, more preferably 5 to 30 µM.

The culture in step (4) is generally performed in a CO₂ incubator at a culture temperature suitable for cell culture (generally 30 to 40°C, preferably about 37°C). The culture period is generally 1 to 3 days, preferably 1 to 2 days.

In step (5), the cells obtained in step (4) are cultured in a medium containing a KGF, a hedgehog signaling inhibitor, and a retinoic acid receptor agonist. In this step, the concentration of the retinoic acid receptor agonist in the medium is set to a concentration lower than the concentration of the retinoic acid receptor agonist in step (4).

Examples of the hedgehog signaling inhibitor to be used in this step include those described in the first method for inducing differentiation into insulin-producing cells, particularly preferably SANT-1.

Examples of the retinoic acid receptor agonist to be used in this step include those described in the first method for inducing differentiation into insulin-producing cells, particularly preferably a retinoic acid.

A medium obtained by adding a KGF, a hedgehog signaling inhibitor, and a retinoic acid receptor agonist to a basal medium can be used as the medium of step (5). The concentration of the KGF in the medium is preferably 10 to 100 ng/mL, more preferably 30 to 80 ng/mL. The concentration of the hedgehog signaling inhibitor in the medium is appropriately set depending on the type to be used but is preferably 0.1 to 0.5 µM, more preferably 0.2 to 0.3 µM, in the case of using SANT-1. The concentration of the retinoic acid receptor agonist in the medium is not specifically limited, as long as it is lower than the concentration of the retinoic acid receptor agonist in step (4), but is preferably 10 to 500 nM, more preferably 50 to 300 nM, for example, in the case of using a retinoic acid. The basal medium to be used may be the same as the medium used in the method for promoting differentiation of pluripotent stem cells of the present invention. Further, the medium of step (5) may contain a serum replacement and other additives like the medium used in the method for promoting differentiation of pluripotent stem cells of the present invention.

The medium of step (5) may contain at least one selected from the group consisting of a BMP signaling inhibitor, vitamin C, Y27632, and an activin receptor-like kinase-4,7 activator.

Examples of the BMP signaling inhibitor to be used in this step include those described in the first method for inducing differentiation into insulin-producing cells, particularly preferably LDN193189.

Examples of the activin receptor-like kinase-4,7 activator to be used in this step include those described in the method for promoting differentiation of pluripotent stem cells, particularly preferably activin A.

The concentration of the BMP signaling inhibitor in the medium is appropriately set depending on the type to be used but is preferably 10 to 500 nM, more preferably 50 to 300 nM, in the case of using LDN193189. The concentration of vitamin C in the medium is preferably 10 to 100 µg/mL, more preferably 20 to 70 µg/mL. The concentration of Y27632 in the medium is preferably 1 to 50 µM, more preferably 5 to 30 µM. The concentration of the activin receptor-like kinase-4,7 activator in the medium is appropriately set depending on the type to be used but is preferably 10 to 500 µg/ml, more preferably 50 to 300 µg/ml, in the case of using activin A.

The culture in step (5) is generally performed in a CO₂ incubator at a culture temperature suitable for cell culture (generally 30 to 40°C, preferably about 37°C). The culture period is generally 3 to 7 days, preferably 4 to 6 days.

In step (6), the cells obtained in step (5) are cultured in a medium containing a TGF-βI type activin receptor-like kinase-4,5,7 inhibitor and a retinoic acid receptor agonist.

Examples of the TGF-βI type activin receptor-like kinase-4,5,7 inhibitor to be used in this step include those described in the first method for inducing differentiation into insulin-producing cells, particularly preferably TGF-βI type receptor-like kinase inhibitor II (ALK5i).

Examples of the retinoic acid receptor agonist to be used in this step include those described in the first method for inducing differentiation into insulin-producing cells, particularly preferably a retinoic acid.

A medium obtained by adding a TGF-βI type activin receptor-like kinase-4,5,7 inhibitor and a retinoic acid receptor agonist to a basal medium can be used as the medium of step (6). The concentration of the TGF-βI type activin receptor-like kinase-4,5,7 inhibitor in the medium is appropriately set depending on the type to be used but is preferably 1 to 50 µM, more preferably 5 to 30 µM, in the case of using TGF-βI type receptor-like kinase inhibitor II. The concentration of the retinoic acid receptor agonist in the medium is appropriately set depending on the type to be used but is preferably 10 to 500 nM, more preferably 50 to 300 nM, in the case of using a retinoic acid. The basal medium to be used may be the same as the medium used in the method for promoting differentiation of pluripotent stem cells of the present invention. Further, the medium of step (6) may contain a serum replacement and other additives like the medium used in the method for promoting differentiation of pluripotent stem cells of the present invention.

The medium of step (6) may contain at least one selected from the group consisting of an EGF receptor agonist, a γ-secretase inhibitor, triiodothyronine, and vitamin C.

Examples of the EGF receptor agonist to be used in this step include those described in the first method for inducing differentiation into insulin-producing cells, particularly preferably an EGF or betacellulin.

Examples of the γ-secretase inhibitor to be used in this step include those described in the second method for inducing differentiation into insulin-producing cells, particularly preferably DAPT or γ-secretase inhibitor XXI.

The concentration of the EGF receptor agonist in the medium is appropriately set depending on the type to be used but is preferably 10 to 50 ng/mL, more preferably 30 to 40 ng/mL, in the case of using an EGF, and is preferably 5 to 100 ng/mL, more preferably 10 to 50 ng/mL, in the case of using betacellulin. The concentration of the γ-secretase inhibitor in the medium is appropriately set depending on the type to be used but is preferably 1 to 50 µM, more preferably 5 to 30 µM, in the case of using DAPT, and is preferably 0.1 to 10 µM, more preferably 0.5 to 5 µM, in the case of using γ-secretase inhibitor XXI. The concentration of triiodothyronine in the medium is preferably 0.1 to 5 µM, more preferably 0.5 to 3 µM. The concentration of vitamin C in the medium is preferably 10 to 100 µg/mL, more preferably 30 to 80 µg/mL.

The culture in step (6) is generally performed in a CO₂ incubator at a culture temperature suitable for cell culture (generally 30 to 40°C, preferably about 37°C). The culture period is generally 2 to 6 days, preferably 3 to 5 days.

In step (7), the cells obtained in step (6) are cultured in a medium containing a TGF-βI type activin receptor-like kinase-4,5,7 inhibitor and a retinoic acid receptor agonist. In this step, the concentration of the retinoic acid receptor agonist in the medium is set to a concentration lower than the concentration of the retinoic acid receptor agonist in step (6).

Examples of the TGF-βI type activin receptor-like kinase-4,5,7 inhibitor to be used in this step include those described in the first method for inducing differentiation into insulin-producing cells, particularly preferably TGF-βI type receptor-like kinase inhibitor II (ALK5i).

Examples of the retinoic acid receptor agonist to be used in this step include those described in the first method for inducing differentiation into insulin-producing cells, particularly preferably a retinoic acid.

A medium obtained by adding a TGF-βI type activin receptor-like kinase-4,5,7 inhibitor and a retinoic acid receptor agonist to a basal medium can be used as the medium of step (7). The concentration of the TGF-βI type activin receptor-like kinase-4,5,7 inhibitor in the medium is appropriately set depending on the type to be used but is preferably 1 to 50 µM, more preferably 5 to 30 µM, in the case of using TGF-βI type receptor-like kinase inhibitor II. The concentration of the retinoic acid receptor agonist in the medium is not specifically limited, as long as it is lower than the concentration of the retinoic acid receptor agonist in step (6), but is preferably 2 to 100 nM, more preferably 10 to 50 nM, for example, in the case of using a retinoic acid. The basal medium to be used may be the same as the medium used in the method for promoting differentiation of pluripotent stem cells of the present invention. Further, the medium of step (7) may contain a serum replacement and other additives like the medium used in the method for promoting differentiation of pluripotent stem cells of the present invention.

The medium of step (7) may contain at least one selected from the group consisting of an EGF receptor agonist, a γ-secretase inhibitor, triiodothyronine, and vitamin C.

Examples of the EGF receptor agonist to be used in this step include those described in the first method for inducing differentiation into insulin-producing cells, particularly preferably EGF or betacellulin.

Examples of the γ-secretase inhibitor to be used in this step include those described in the second method for inducing differentiation into insulin-producing cells, particularly preferably DAPT or γ-secretase inhibitor XXI.

The concentration of the EGF receptor agonist in the medium is appropriately set depending on the type to be used but is preferably 10 to 50 ng/mL, more preferably 30 to 40 ng/mL, in the case of using an EGF, and is preferably 5 to 100 ng/mL, more preferably 10 to 50 ng/mL, in the case of using betacellulin. The concentration of the γ-secretase inhibitor in the medium is appropriately set depending on the type to be used but is preferably 1 to 50 µM, more preferably 5 to 30 µM, in the case of using DAPT, and is preferably 0.1 to 10 µM, more preferably 0.5 to 5 µM, in the case of using a γ-secretase inhibitor XXI. The concentration of triiodothyronine in the medium is preferably 0.1 to 5 µM, more preferably 0.5 to 3 µM. The concentration of vitamin C in the medium is preferably 10 to 100 µg/mL, more preferably 30 to 80 µg/mL.

The culture in step (7) is generally performed in a CO₂ incubator at a culture temperature suitable for cell culture (generally 30 to 40°C, preferably about 37°C). The culture period is generally 1 to 5 days, preferably 2 to 4 days.

### Examples

Hereinafter, the present invention will be specifically described further in detail by way of examples, but the present invention is not limited to these examples.

### (A) Results

### (1) Methionine deprival pretreatment potentiates late stage differentiation of human iPS cells into insulin-expressing beta cells.

The inventor previously reported that pluripotent stem cells showed a special metabolic state that requires methionine metabolism. Deprival of methionine from media rendered the pluripotent stem cells at a biased state for differentiation, so that if inductive signaling was applied, a potentiated differentiation was observed.

The inventor then investigated if methionine pretreatment could potentiate late stage differentiation into pancreatic beta cells.

The inventor differentiated Toe hiPSCs using the previously reported procedure for generating insulin-expressing beta cells with some modifications (Nakashima et al., 2015; Shahjalal et al., 2014). For differentiation into pancreatic endocrine cells, the inventor dissociated undifferentiated hiPSCs and cultured under pluripotent cell maintenance medium to allow the cells to form spheres for 24 h.

After formation of spheres, hiPSCs were treated 5 h with the same media that was deprived of methionine (ΔMet, or contr), then triggered to differentiate by switching to differentiation media (Figure 1A). This protocol for initiation of differentiation and treatment of methionine deprival is used throughout this study. The morphologies of the differentiated cells on day 3 and day 13 were similar between the two groups but after day 25, differences became evident between the two groups: large spheres appeared in control but those in ΔMet treated group were of uniform sizes (Figure 1B).

The inventor compared gene expressions between the methionine deprival treated and control groups at different differentiation stages by performing microarray analysis (Figures 1C and 1D). Primary human pancreatic islets (islets) were included as a control. Principal component analysis (PCA) of gene expressions with the highest variance revealed that differences between ΔMet (red) and complete (blue) are not so large at definitive endoderm (DE) or primitive gut (PG) stage, but increased along PC1 in day 13 (D13) and 25 (D25) differentiated cells (Figure 1C). Analysis on individual gene expressions in hiPSC-derived cells revealed that differentiation and maturation markers increased in D11 pancreatic progenitor (PP), D13 endocrine progenitor (EP) and D25 endocrine cell (EC) spheres pre-treated with ΔMet compared to those of the controls (Figure 1D).

Immunocytochemistry analysis of the differentiated cells revealed that the proportion of OCT3/4-expressing cells were significantly reduced in differentiation day 3 DE cells in ΔMet treated groups (Figure 1E). The increase in SOX17-positivity was not evident, which might due to a high SOX17 positivity in control complete medium treated condition. In ΔMet treated cells, C-peptide positive cells significantly increased in differentiation day 19 early EC cells (Figure 1F), and increased thereafter, and the proportion of C-peptide positive cells in differentiation day 25 EC cells (Figure 1G) reached approximately 50%. Methionine deprival protocol was also be able to apply to another hiPS cell line, RPChiPS771, which was established using a synthetic self-replicative RNA (Yoshioka et al., 2013). Compared to the control untreated cells, methionine deprival treated cells showed a potentiated differentiation with a decreased proportion of OCT3/4-positive cells on differentiation day 3 (Figure 1H), and an increased proportion of INS+/PDX1+ cells on differentiation day 25 (Figure 1I). Compared to the control untreated cells, the resultant iPS-derived beta cells showed improvement in the ability of glucose-stimulatory insulin secretion (GSIS), so that the high-to-low glucose ratio is increased compared to the control complete medium treated cells, and therefore is responsive to high glucose similar to cells of the human primary pancreatic islets tested (Figure 1J).

### (2) Methionine deprival pretreatment potentiates differentiation.

Next, the molecular mechanism that methionine deprival potentiated differentiation will be revealed. Human RPChiPS771 cells were subjected to amino acids singly deprival and then differentiated into DE under two different protocols (Figures 2A and 2B). In protocol A, where subsequent differentiations were done without CHIR99021, yielded high proportions of SOX17+ cells particularly under ΔMet pretreatment. In protocol B, in which CHIR99021 was added, a higher proportion of SOX17+ cells overall, and even higher SOX17+ under ΔMet treatment was observed. Threonine deprival also revealed a significant increase in SOX17+ cells in either with or without CHIR99021, but at a less degree than methionine deprival (Figures 2A and 2B).

To explore into the molecular mechanism of the potentiation of differentiation, the inventor analyzed gene expression profiles of the human iPSCs after treated with short term (5h) methionine or other amino acid deprival. In this experiment, RPChiPS771 and 201B7 hiPSCs were used. Gene expressions were analyzed by real time PCR and PCA, using a primer set consisting of 88 known genes related to pluripotency and differentiation, together with 8 housekeeping genes (Figure 2C). PCA revealed that methionine deprival of the pluripotent stem cells (Figure 2D, closed red circles) showed a distinct expression profile from those of complete medium (Figure 2D, closed blue circles) or other amino acid deprival (Figure 2D, closed grey circles). Genes upregulated or down regulated are shown as a heat map in Figure 2E. Of the genes that were down-regulated or upregulated in both RPChiPS771 and 201B7 hiPSCs, the inventor noticed that a pluripotency marker NANOG (Mitsui et al., 2003) is down-regulated upon methionine deprival. By contrast, an endodermal gene GATA6 that was recently reported to have a role in endoderm differentiation (Shi et al., 2017), a mesodermal marker gene COL1A1, and an ectodermal marker gene PAX6, are up-regulated under methionine deprival in both hiPSCs. The results suggest that undifferentiated hiPSCs exhibited changes in gene expression so that the cells are biased toward differentiation. The present results explain the cells pretreated with methionine deprival showed a potentiated differentiation into three germ layer.

### (3) SLC30A1 expression is up-regulated under methionine deprivation.

To explore into the gene that are upregulated upon methionine deprival of the pluripotent stem cells for short term (5h), human khES3 ESCs and 201B7 hiPSCs were cultured in complete or methionine deprived CSTI7 or StemFit serum free medium for 5 h and RNA was extracted and subjected to gene array profiling analysis, using 3 different array sets (Figure 3A). Nine genes that were observed to be upregulated in all three experiments are listed in Figure 3B. One of the genes that upregulated in methionine deprival treated cells was SLC30A1 gene that encodes ZnT1 protein (Andrews et al., 2004), a Zn efflux transporter that localized to the plasma membrane. SLC30A1 is a member of the solute-linked carrier (SLC) gene family that promote zinc efflux from cells or into intracellular vesicles and reduce intracellular Zn. The inventor then re-examined the array analysis data of the genes SLC30A2-10 and SLC39A1-14, members of the solute-linked carrier gene family encoding ZIP proteins that promote intake of Zn to increase intracellular Zn. As a result, several members of the SLC30A or SLC39A were revealed to be expressed in the undifferentiated and differentiated hiPSCs in the array analyses by the inventor (Figure 3C). The inventor then focused on the expression of SLC30A and SLC39A gene family members expressed in the hiPSCs and examined if their expression levels are specifically affected by methionine or other amino acid short term (5h) deprival, using RPChiPS771 cells. As a result, SLC30A1 was specifically upregulated (> 6-fold) under methionine deprival but not other amino acid deprival. SLC39A1 was also slightly upregulated (> 1.5-fold) by methionine deprival (Figure 3D). (4) Reduced protein bound Zn under methionine deprival might explain the biased state for differentiation triggered by methionine deprival treatment of pluripotent hiPSCs.

SLC30A1 encodes ZnT1 which is expressed in the plasma membrane, and functions to excrete Zn and decrease intracellular Zn contents. The inventor further measured heavy metals, Zn, Cu and Fe contents (labile or protein bound forms), in the pluripotent stem cells treated with methionine deprival or other amino acid deprival for 5 h (Figure 4A). As a result, a reduction in intracellular protein bound Zn was specifically observed upon methionine deprival but not other single amino acid deprival (Figure 4A).

The inventor then hypothesized that a reduced intracellular Zn concentration caused by methionine deprival might explain its effects to trigger a differentiation of pluripotent stem cells. To test this possibility, the inventor then attempted to reduce intracellular Zn concentration by culturing pluripotent hiPSCs in Zn deprived media. Maintenance medium StemFit AK medium were deprived of Zn by treating with a chelating reagent, Chelex 100. Since Chelex 100 not only chelates Zn but also other heavy metals, such as copper, iron, magnesium, and calcium from the medium, other heavy metal ions were added back to make Zn deprived medium (ΔZn, Figure 4B). Pluripotent iPS cells were then cultured in Chelex (ΔZn) treated media for 24 h, 48 h, and 72 h, and then assayed for cell proliferation and intracellular Zn concentrations.

Under control complete medium, cell numbers increased with time. However, when cultured under ΔZn condition, cell numbers reduced, and showed minimum increase at 72 hr, which is significantly lower than that of the control complete condition (Figure 4C, D). Intracellular protein bound Zn contents decreased significantly under ΔZn condition at 48 hr and further at 72 hr (Figure 4E). The results therefore suggest that culturing under ΔZn condition triggered a gradual decrease in intracellular Zn contents, and led to a cessation of cell proliferation. Thereafter, gene expressions were analyzed by real time PCR using the same primer array set described above. Pluripotent stem cells cultured under ΔZn condition showed reduced expressions of LIN28, DNMT3A, TERT (encoding telomerase reverse transcriptase) and ZFP42 (a zinc finger protein also known as REX2), which are markers important to maintain the pluripotent state of the iPSCs (Figure 4F). Increased expressions of EOMES (encoding Eomesodermin, or T-box brain protein 2), SOX17, CDH5, GATA4 and ISL1 that mark differentiated cells (Figure 4G) were also observed.

To compare the affected genes between iPSCs treated with ΔZn and ΔMet, the expression of genes in pluripotent stem cells cultured under ΔMet condition was also examined using the same primer arrays used in Figures 4F and 4G. It revealed that the expression of the above mentioned genes in iPSCs also show similar tendency compared to those cultured under complete media, namely, the genes that mark pluripotency states, such as DNMT3B, TERT and ZFP42 were down regulated, and the genes that mark differentiated cells, such as EOMES, SOX17, GATA4 and ISL1 were up regulated. The results therefore suggest that decreased intracellular Zn mimicked methionine deprival and increased the potency of differentiation. Taken together, the results by the inventor strongly suggest that reducing intracellular Zn might be a downstream target of methionine deprival (Figure 4H).

### (5) Roles of Zn in maintenance of undifferentiated iPS cells

The results by the inventor suggest that SLC30A1 (ZnT1)-mediated reduction of intracellular Zn might be one of the mechanisms that underlies potentiated differentiation through ΔMet treatment. Since ΔMet treatment affected the maintenance of pluripotent stem cells (Shiraki et al, 2014), it was then investigated if reduced Zn concentration in the media might affect the growth or pluripotency of the undifferentiated pluripotent stem cells.

Insulin solution contains Zn as a stabilizer and is often used as a supplement in the media to sustain self-renewal of undifferentiated iPSCs. The use of insulin as a supplement in the medium resulted Zn at 3 µM final concentration in StemFit AK03N. To allow manipulation of Zn content in the medium, the inventor deprived Zn from StemFit AK03 [Undiff-AKM (INS/Zn0)] and substituted insulin with IGF1 [Undiff-AKM (IGF1/Zn0)]. For maintenance culture of pluripotent hiPSCs, the Zn concentration was adjusted to 3 µM (Figure 5A).

To confirm that IGF1 could sustain pluripotency of hiPSCs, hiPSCs were cultured in medium supplemented with insulin or IGF instead of insulin, and primer array analyses were performed, to compare their expressions using a primer array. The undifferentiated iPSCs cultured in undiff-AKM (INS) (Zn3) (= StemFit AK03N) or undiff-AKM (IGF1) (Zn3) showed similar expression profiles, in contrast to those shown in the differentiated cells of day 1 or day 3 DE cells (Figure 5B). The results therefore indicate that hiPSCs cultured at the presence of IGF1 are not different from those cultured in conventional media containing insulin.

Using this undiff-AKM (IGF) medium, the effects of Zn concentrations on the growth of iPSCs were then examined. Undifferentiated Ff-I01s01 cells were cultured for 3 days in maintenance media undiff-AKM (IGF1) or containing 6 different Zn concentrations, and then their viabilities were examined. iPSCs cultured under 0 µM Zn showed a sharp decrease of cell numbers (Figures 11A and 11B). Cells cultured under 0 µM Zn showed cell death that became obvious from day 2. While some dead cells also can be observed in 0.5 µM Zn cells, cell proliferation is confirmed under conditions of 0.5, 1, 3, 10 µM Zn (Figure 11A). To allow studies on the effects of IGF1, the inventor further compared the used of IGF1 with a fast acting insulin analogue, insulin glulisine, containing no Zn binding sites (INS*), by real time PCR analysis of the undifferentiated iPSCs. On day 3, iPSCs were analyzed for expression of genes associated with pluripotency maintenance or differentiation. Undifferentiated iPSCs cultured under 0, 0.5 and 1 µM Zn media in AKM (IGF1) showed down-regulation of genes associated with pluripotency, such as NANOG, OCT3/4, compared to those cultured in AKM (IGF1) at 3 µM Zn. Genes associated with cell growth, such as DNMT3, FGF7, CCL2, CRABP2, HCK (hemopoietic cell kinase) and GRB7 (growth factor receptor-bound protein 7), similarly showed decreased expressions in those cultured at 0 µM Zn compared to 3 µM Zn in AKM (IGF1) (Figure 11C). The results were more consistent under AKM (IGF1) than AKM (INS*) condition.

For the effects on differentiation markers, most differentiation markers tested were up-regulated, such as GATA4 (an endoderm marker), PECAM (platelet endothelial cell adhesion molecule; a mesoderm marker), EOMES, MNX1, PAX6 (paired box protein; an ectoderm marker) in cells cultured at 0 µM Zn in AKM (IGF1) or AKM (INS*) compared to controls 3 µM Zn in AKM (IGF1) (Figure 11D). In contrast, expression of a differentiation marker, LEFTY1, increased specifically under 3 µM Zn in AKM (INS*) condition (Figure 11E).

The results therefore suggest that iPS cells cultured at 0 µM Zn in AKM (IGF1) condition showed decreased cell proliferation and potentiated differentiation (Figure 11). Insulin signaling seems to exert strong effects to increase several expressions of pluripotency or cell growth markers, even under 0 µM Zn conditions.

### (6) Deprival of Zn from differentiation media potentiated differentiation into DE cells.

For differentiation, in order to determine an appropriate Zn concentration, AKM media deprived of Zn (AKM Zn0) and adjusted Zn concentration at 0, 0.5 or 3 µM were used. The concentrations of Zn in the media used in this study are shown in Figure 5A.

8 hiPSC lines derived from HLA homozygote donors (Sugita et al., 2016) were then differentiated into the DE cells using the xeno-free StemFit AKM medium as differentiation medium M1, in which insulin is supplemented (M1-AKM (INS)) (Figure 5C). However, immunocytochemical analysis revealed that a substantial proportion of cells retained OCT3/4 expression (Figure 5D). The inventor interpreted this to mean that Zn concentration at 3 µM might be not suitable for differentiation. The inventor then used Ff-I01s01, Ff-I01s04, Ff-I14s03, Ff-I14s04, Ff-MH09s01, Ff-MH15s01, Ff-MH15s02, and Ff-MH23s01 iPSCs and tried to reduce Zn in M1. With these hiPSC lines, differentiation at the presence of IGF1 instead of insulin and removal of Zn from the basal media M1-AKM (IGF1/ Zn 0) effectively reduced OCT4+ cells (Figure 5E).

### (7) Deprival of Zn and methionine potentiated differentiation into a pancreatic fate.

The inventor further tested Zn concentration dependency in differentiation into a pancreatic fate, in combination with methionine deprival pretreatment. Figure 6A shows the xeno-free culture procedure of the present invention used for pancreatic differentiation.

The inventor used Ff-I01s01 iPSCs and cultured under methionine deprival for 5 h before differentiation. Zn concentration at 0, 0.5 or 3 µM in M1-AKM (IGF1) medium was tested for differentiation into day 3 DE cells. Under complete media, OCT3/4+ DE cells appeared at Zn 0.5 µM and increased at Zn 3 µM, with a reciprocal decrease in SOX17+ cells. Upon ΔMet treatment applied before differentiation, OCT3/4+ cells were eliminated at either Zn 0, 0.5 or 3 µM, and a reciprocal increase in SOX17+ cells was observed (Figure 6B, upper panel). Further differentiation of DE cells into day 13 EP cells revealed that without ΔMet treatment, PDX1-positive cells were highest when M1 differentiation medium contained 0.5 µM Zn (IGF1/Zn 0.5). By contrast, when M1 contains IGF1/ Zn0 or IGF1/Zn 3, a decreased PDX1 proportion was observed. However, when ΔMet treatment was applied before differentiation, differentiation into PDX1-positive cells increased even at Zn 3 µM. These results therefore suggest that Zn concentration in M1-AKM is important for the pancreatic differentiation, and that ΔMet could enhance differentiation.

Another HLA homozygote-derived iPSC line, Ff-I14s04, was also tested (Figure 13). Ff-I14s04 cells were differentiated in M1-AKM (IGF1) or M1-AKM (INS*) at the presence of Zn concentration at 0, 0.5 or 3 µM. In this experiment, insulin glulisine, a fast acting insulin analogue that contains no Zn was used. On day 3, DE cells were assayed for SOX17 or OCT3/4-positivities. The results revealed that at the absence of Zn during M1 (AKM (IGF1/Zn0)), a significant decrease in OCT3/4-positive cells and increase in SOX17-positive cells was observed. Increasing Zn to 3 µM increased OCT3/4 positive cells. When IGF1 was substituted with INS*, OCT3/4 increased even at Zn concentration 0 µM, and to a higher level at Zn concentration 3 µM, with a concomitant decrease in SOX17-positive (Figure 13B). Subsequent pancreatic differentiation into PDX1+ cells decreased when M1-AKM was conducted at 3 µM Zn concentration, or even lesser when INS* was used at 0 µM or 3 µM Zn concentration. These results revealed an unexpected role of insulin signal or Zn to sustain OCT3/4 expression and inhibit endoderm differentiation. The combinatory action of INS and Zn is a strong signal to sustain OCT3/4 expression and inhibit endoderm and further pancreatic differentiation, which is consistent with the results of Figure 11 above.

To optimize Zn concentration for endoderm differentiation, HLA homozygote-derived iPSC line Ff-I01s01 cells were differentiated in M1-AKM (IGF1), M1-AKM (IGF2), M1-AKM (INS*), or control M1-AKM (INS) [Basic03 (INS_3)], and added with a graded dose of Zn concentration (0, 0.5, 1, 3 µM) for each day during the 3 day time (Figure 12A).

Expression of genes associated with pluripotency, such as POU5F1, SOX2, NANOG or GBX2, a gene associated with cell growth, were down-regulated when M1-AKM (IGF1) or M1-AKM (IGF2) were used compared to control M1-AKM (INS) [Basic03 (INS_3)] (Figure 12B-E). Compared to cells cultured under Zn deprived condition for 3 days M1-AKM [IGF1 (0_0_0)], M1-AKM [IGF1 (3_3_3)] or M1-AKM [IGF1 (05_1_3)] showed a significant although small increase in the expression of the pluripotency markers or cell growth marker (Figure 12b-e). The results therefore indicate that INS exhibit strong activity in the maintenance of pluripotency and sustaining the growth of undifferentiated hiPS cells. Moreover, > 1 µM Zn concentration significantly increased the growth of hiPS cells.

By contrast, expressions of differentiation markers such as CD34 (mesoderm), PAX6 (neural ectoderm) and GATA6 (definitive endoderm) were up-regulated when M1-AKM [IGF1 (3_3_3)] was used compared to control M1-AKM (INS) [Basic03 (INS_3)]. Lowering Zn concentration itself did not upregulated these differentiation markers when INS* was used. Down-regulation of CD34 or PAX6 expression was observed with increased Zn concentrations in M1-AKM (IGF1, IGF2) compared to M1-AKM [IGF1 (0_0_0)].

Taken together, it was found that INS exhibit a strong pluripotency maintenance activity, and Zn concentration also acts to sustain the pluripotency. With the use of INS and increased Zn concentration, the pluripotency maintenance and the growth of iPS cells is sustained.

Then, using a spinner flask for a large scale culture of iPSC-derived pancreatic cells, the effects of ΔMet treatment in combination with culturing under M1-AKM (IGF1/Zn 0.5) condition were tested to yield differentiation day 11 pancreatic PP and then differentiation day 22 EC cells, following the procedure shown in Figure 6A. Immunocytochemical analysis of PDX1-positivity revealed that under this culture condition, differentiated cells on day 11 yielded 83 or 87% PDX1-positivity upon complete or ΔMet conditions, respectively (Figure 6D). However, later on in differentiation day 22 EC cells, approximately 21% INS +NKX6.1+ double-positive cells were observed in ΔMet treated cells, but only 3% INS+ cells were obtained in those grown under control complete condition (Figure 6E). The results suggest that Zn at early stage is required for the differentiation into INS +NKX6.1+ double-positive cells. ΔMet and 0.5 µM Zn act to potentiate differentiation into PDX1-positive EC cells.

Taken together, the results by the inventor indicate that ΔMet treatment potentiated differentiation, and one of the downstream targets is up-regulation of Zn transporter ZnT1, which then decreases intracellular Zn contents. Decreased Zn content during initial differentiation potentiated differentiation into DE cells and mimicked ΔMet. However, a decreased Zn is not sufficient for an efficient differentiation into a pancreatic fate, and application in combination with ΔMet treatment potentiated pancreatic differentiation.

### (8) Deprival of Zn and methionine potentiated differentiation into beta cells.

The inventor then attempted to adapt the ΔMet ΔZn novel condition to previously described protocols (Pagliuca et al, 2014; Ve). The experimental scheme is shown in Figures 7A and 7B (also see Material and methods). The inventor conducted 2 conditions for differentiating into pancreatic endocrine beta cells (Figure 7B), in which M1 and S2 steps are modified. The morphologies of the differentiated cells at indicated stages are shown in Figure 7C. Immunocytochemical analysis revealed that the two conditions gave similarly high SOX17-positivity at day 3 DE (Figure 7D). At the EP (endocrine progenitor) (day 14) stage, NKX6.1-positive cells appeared, and PDX1+/NKX6.1+ double positive cells increased in conditions a and b. Then at EC (endocrine cell) stage, insulin cells increased, in which PDX1+/NKX6.1+/INS+ triple positive cells were high in conditions a and b (Figure 8A).

Glucose-stimulatory insulin secretion assay was then performed to test the functionality of the differentiated cells. The results revealed that conditions a and b showed GSIS activity. Of note is that condition a showed a clear biphasic GSIS activity with fast responding phase 1 of insulin secretion on day 26. Immunocytochemical analysis on day 33 revealed that the cells are positive for insulin, NKX6.1 and MAFA in conditions a and b in insulin staining.

The results by the inventor revealed that the methionine deprival and low Zn procedure are applicable for the generation of pancreatic endocrine beta like cells that are capable of GSIS activity. Studies on the 2 conditions confirmed that low RA and vitamin seem to be required for the survival of the differentiated cells, and therefore help to optimize the condition for differentiation into functional beta like cells.

The inventor then attempted to adapt the ΔMet ΔZn novel condition to previously described protocols (Pagliuca et al, 2014; (Velazco-Cruz et al., 2018). The experimental scheme is shown in Figures 9A and 9B (also see Material and methods). The inventor conducted 2 conditions for differentiating into pancreatic endocrine beta cells (Figure 9A), in which S3, S4, S5-1 and S5-2 steps are modified. The cells yielded from conditions #1 and #2 were then cultured in 8 or 6 conditions shown in Figure 9B, respectively. Immunocytochemical analysis revealed that the two conditions gave similarly high SOX17-positivity at day 3 DE (Figure 9C), and high PDX1+ or SOX9+ pancreatic progenitor cells at day 7 pancreatic progenitor (PP) stage (Figure 9D). On day 12 at endocrine progenitor (EP) stage, NKX6.1+ cells appeared, and PDX1+ or NKX6.1+ positive cells increased in both conditions (Figure 9E). Then on day 19 at endocrine cell (EC) stage, insulin cells increased, in which NKX6.1+/INS+ double positive cells reached approximately 20% in both conditions (Figure 10A).

Then the yielded cells were cultured in 8 or 6 different conditions in S6. At the end of S6, glucose-stimulatory insulin secretion assay was then performed to test the functionality of the differentiated cells, under condition #1 (Figure 10B) or condition #2 (Figure 10C), cultured under these different S6 conditions. The results revealed that in both conditions #1 and #2, cells cultured particularly under conditions a-2, c and d, and A-3 yielded cells that showed a high GSIS activity. Under these conditions, the iPS-derived beta cells responded rapidly to high glucose and secreted C-peptide within 10 min exposure, which lasted until 60 min or longer. Of note is that a clear biphasic GSIS activity with fast responding phase 1 of insulin secretion was observed in cells cultured under conditions #1 and c.

Immunocytochemical analysis on day 30 revealed that the cells are positive for insulin, NKX6.1 and MAFA. Conditions a-2, c, d and A-3 under #1 and #2 gave rise to Cpep+ cells that approximately half of the Cpep+ cells coexpressed NKX6.1 or MAFA (Figures 10D, 10E, 10F, and 10G). These results revealed that the methionine deprival and low Zn procedure of the inventor are applicable for the generation of pancreatic endocrine beta like cells that are capable of GSIS activity.

### (B) Material and methods

### (1) Human iPS cell lines

Toe human iPS cell (hiPSC) line was established by Toyoda M., Kiyokawa N., Okita H., Miyagawa Y., Akutsu H. and Umezawa A. in National Institute for Child Health and Development, Tokyo. RPChiPS771 cells were obtained from REPROCELL Inc. (Tokyo) (Yoshioka et al, CSC 201). 201B7, HLA homozygote donor derived iPSCs, Ff-I01s01, Ff-I14s03, Ff-I01s04, Ff-I14s04, Ff-MH09s01, Ff-MH15s01, Ff-MH15s02 and Ff-MH23s01, were obtained from Center for iPS Cell Research and Application, Kyoto University (CiRA).

### (2) Maintenance culture of undifferentiated hiPSCs

Undifferentiated Toe and RPChiPS771 cells were maintained in StemFit AK02N media (Ajinomoto Co., Inc., Tokyo), and HLA homozygote donor derived iPSCs were maintained in StemFit AK03N. StemFit media were supplemented with penicillin and streptomycin. Human iPSCs were freeze thawed and cultured on 100 mm CellBIND dish (Corning Incorporated) pre-coated overnight with Synthemax II (Corning Incorporated, Cat No. 3535) in maintenance media supplemented with 10 µM Y27632 (FUJIFILM Wako Pure Chemical Corporation, Cat No. 251-00514) .

### (3) Medium

StemFit KA01 medium is methionine-deprived medium of StemFit AK media. StemFit (registered trademark) Basic03 is Stem Fit AK03N without component C. If not else indicated of Zn concentration, AKM medium is Zn deprived, insulin deprived medium of StemFit Basic03.

AKM (INS) medium is AKM medium + 100 ng/mL insulin.

AKM (IGF) medium: AKM medium + 100 ng/mL IGF1 (to substitute insulin).

AKM (INS*) medium is AKM medium + 100 ng/mL insulin glulisine (Apidra (registered trademark)).

Undiff-AKM medium is AKM medium + component C.

### (4) Three dimensional sphere formation, methionine deprivation pretreatment

Undifferentiated hiPSCs cultured on two dimensional plates pre-coated with SynthemaxII were dissociated, and replated onto 6-well suspension culture plates at a density of 5x10⁶ cells/well (Greiner Bio-One International GmbH, #657185), and cultured at 37°C for 24 h to form sphere on an rotating orbital shaker at 95 rpms. Then, methionine deprivation pretreatment was done by culturing the cells for 5 h in Met-deprived StemFit KA01 medium or control complete media (StemFit KA01 +100 µM methionine) (Ajinomoto Co., Inc., Tokyo), before initiation of differentiation.

### (5) Cell viability assay and RT-PCR analysis with undifferentiated iPS cells cultured in media containing various Zn concentrations

Undifferentiated Ff-I01s01 cells were thawed and cultured in AK03N. After reaching 80-90% cell confluency, the cells were plated 2000 cells/well in 96-well plates in AK03N. On the next day, the media were switched to AKM (IGF1) at 6 different Zn concentrations + component C, and further cultured for 3 days, then assayed for cell viability using PrestoBlue (trademark) Cell Viability Reagent (Invitrogen). For real time PCR analysis, undifferentiated Ff-I01s01 cells were seeded at 1 x 10⁵ cells/well in a 6-well plate in maintenance media AK03N. On the next day, the media were changed to AKM (IGF1) at 5 different Zn concentrations, or AKM (INS*; Apidra, an insulin analogue with no Zn binding sites) at 3 different Zn concentrations + component C, then cultured for 3 days. On day 3, cells were collected, and mRNAs were extracted and subjected for real time PCR analyses using a PrimerArray (registered trademark) Embryonic Stem Cells (Takara Bio Inc.).

### (6) Differentiation of iPS cells into pancreatic lineage cells

Initially, the inventor used the following protocol for differentiation (Figure 1). Initiation of differentiation was done after 5 h complete or methionine deprivation pretreatment. The media used for differentiation of Toe and RPChiPS771 hiPSCs are as followings.

The media used for the 5-stage β cell differentiation basically followed protocol that reported by Shahjalal et al. and Nakashima et al, with minor modifications.
Medium 1 (M1): Initiation of differentiation was done by replacing the culture with Medium 1, including DMEM with 4, 500 mg/L glucose supplemented with 3 µM CHIR99021 (Stemgent), 100 ng/mL recombinant human activin-A (Cell Guidance Systems Ltd), and 2% B27 insulin (+) supplement (Life Technologies). On days 1 and 2, the media were replaced with medium 1 without CHIR99021.
Medium 2 (M2): On days 3 and 4, the media were replaced with Medium 2, containing RPMI (Life Technologies) supplemented with 0.25 µM SANT-1 (Sigma-Aldrich Co. LLC), 50 ng/mL recombinant human FGF10 (Pepro Tech, Inc.) and 2% B27 insulin (-) supplement (Life Technologies).
Medium 3 (M3): From day 5, the media were replaced with Medium 3, containing DMEM with 4, 500 mg/L glucose supplemented with 0.25 µM SANT-1, 0.1 µM LDN193189 (Sigma-Aldrich Co. LLC), 2 µM retinoic acid (Stemgent), and 1% B27 insulin (+) supplement.
Medium 4 (M4): From day 11, the media were changed to Medium 4, including DMEM with 4, 500 mg/L glucose supplemented with 0.1 µM LDN193189, 5 µM TGF-β type I receptor kinase inhibitor II (FUJIFILM Wako Pure Chemical Corporation), 0.3 µM (-)-indolactam V (Sigma-Aldrich Co. LLC), and 1% B27 insulin (+) supplement.
Medium 5 (M5): From day 13 to day 25, cells were cultured in Medium 5, containing Knockout DMEM/F-12 (Life Technologies) supplemented with 50 ng/mL exendin-4 (Cell Sciences, Inc.), 10 mM nicotinamide (Sigma-Aldrich Co. LLC), 1 mM N-acetyl-L-cysteine (NAC) (NACALAI TESQUE, INC.), 10 µM ZnSO₄ (Sigma-Aldrich Co. LLC), and 1% B27 insulin (+) supplement. From day 5, media were replaced every 2 days with fresh media and growth factors.

All media (Media 1 to 4) were supplemented with Penicillin-Steptomycin (PS; NACALAI TESQUE, INC.), MEM Non-essential amino acid (NEAA; Life Technology), and 2-Mercaptoethanol (2-ME; Sigma-Aldrich Co. LLC).

For differentiation of hiPSC from HLA homozygote donors, the media used for differentiation are as following. (version 0) (Figure 6)
Medium 1 (M1-AKM): Differentiation was initiated by replacing the culture with Medium 1, including StemFit AKM (Zn 0.5) (Ajinomoto Co., Inc.) supplemented with 0.5, or 3 µM ZnSO₄, 3 µM CHIR99021 (Stemgent), 100 ng/mL recombinant human activin-A (Cell Guidance Systems Ltd), 100 ng/mL recombinant human IGF1 (Cell Guidance Systems Ltd). The AKM medium is StemFit AK03N medium without Supplement C. StemFit AKM (Zn0) is StemFit AKM medium further deprived of Zn.
Medium 2 (M2-AKM): On days 3 and 4, the media were replaced with Medium 2, containing StemFit AKM (Zn 0) supplemented with 0.5 µM ZnSO₄, 0.25 µM SANT-1 (Sigma-Aldrich Co. LLC), 50 ng/mL recombinant human FGF10 (Pepro Tech, Inc.).
Medium 3 (M3-AKM): From day 5 to day 11, the media were replaced with Medium 3, containing StemFit (registered trademark) Basic03, 25 µM SANT-1, 0.1 µM LDN193189 (Sigma-Aldrich Co. LLC), 2 µM retinoic acid (Stemgent), 44 µg/mL vitamin C (Sigma-Aldrich, Cat No. A4544), and 10 µM Y27632.
Medium 4 (M4-AKM): From day 11 to day 12, the media were replaced with Medium 4, containing StemFit (registered trademark) Basic03 (Ajinomoto Co., Inc.) supplemented with 0.1 µM LDN193189, 5 µM TGF-β type I receptor kinase inhibitor II (FUJIFILM Wako Pure Chemical Corporation), 0.3 µM (-)-indolactam V (Sigma-Aldrich Co. LLC), 100 ng/mL recombinant human EGF (Oriental Yeast Co., ltd.), 10 mM nicotinamide (Sigma-Aldrich Co. LLC), 44 µg/mL vitamin C and 1 µM T3 (Sigma-Aldrich Co. LLC Cat No. T6397).
Medium 5 (M5-AKM): From day 13 to day 22, cells were cultured in Medium 5, containing StemFit (registered trademark) Basic03 supplemented with 50 ng/mL exendin-4 (Cell Sciences, Inc.), 10 mM nicotinamide, 1 mM N-acetyl-L-cysteine (NAC) (NACALAI TESQUE, INC.), 10 µM ZnSO₄ (Sigma-Aldrich Co. LLC), 44 µg/mL vitamin C, 10 µM TGF-β type I receptor kinase inhibitor II and 1 µM T3.

### (7) Single amino acid deprivation

Custom made StemFit AK01 media with each amino acid deprived singly were used (Ajinomoto Co., Inc., Tokyo).

### (8) Zn deprivation from the medium using Chelex 100

Chelex 100 resin was prewashed following manufacturer's instructions (Bio-Rad Laboratories, Inc.). Human iPSC maintenance media 40 ml were added with prewashed Chelex 100 resin 1.2 g and incubated for 2 hr 45 min with rotation, and were filtered (0.22 µm) and quantified the metals by Ajinomoto Co., Inc. Control complete media was made by adding back all heavy metals, ZnSO₄ 7H₂O (Sigma-Aldrich Co. LLC), Fe(NO₃)₃ 9H₂O (FUJIFILM Wako Pure Chemical Corporation, Tokyo), CaCl₂ (NACALAI TESQUE, INC., Kyoto), and MgSO₄ (Sigma-Aldrich Co. LLC). Zn deprived medium was made by adding back the heavy metals excluding only ZnSO₄ 7H₂O.

201B7 undifferentiated hiPSCs were dissociated and plated at 1x10⁵ cells/well on SynthemaxII coated plates 6-well plates (Corning Incorporated) and cultured at 37°C for 24 h. The cells were then washed with PBS (-) and with the medium replaced with Zn deprived or control complete medium described above, and cultured for 24 h, 48 h or 72 h, and then the cells were dissociated, cell counted and harvested.

### (9) A revised protocol for pancreatic differentiation (version 1) (Figures 7 and 8)

The inventor tested the previously published protocol (Pagliuca et al, 2014) using the Met, Zn deprival media AKM (IGF1/Zn0.5) or AK03Basic03 for pancreatic differentiation. The media used were as followings.

Differentiation was initiated by replacing maintenance media with M1-AKM. On day 3, media were replaced with M2-AKM. On day 5, the media were replaced with S2-2 media and cultured for 2 days (or skipped culturing in S2 media). Then on day7 switched to S3 media and cultured for 2 days. Then on day 9, switched to S4 media. On day 14 switched to S5-1 media, and on day 17 switched to S5-2 media. On day 21 switched to S6 media. Medium components are as followings. S2-2 medium: StemFit (registered trademark) Basic03 (FitB) + 50 ng/ml KGF (FUJIFILM Wako Pure Chemical Corporation) and 44 µg/ml vitamin C.
S3 medium: StemFit (registered trademark) Basic03 + 50ng/ml KGF, 0.25 µM SANT, 50 nM indolactam V, 2 µM retinoic acid (RA) and 44 µ/ml vitamin C. S4 medium: StemFit (registered trademark) Basic03 + 50 ng/ml KGF, 0.25 µM SANT, 100 nM RA, 44 µg/ml vitamin C and 100 nM LDN193189.
S5-1 medium: StemFit (registered trademark) Basic03 + 10 µM Alk5i II, 33.3 ng/ml EGF, 10 µM DAPT (FUJIFILM WAKO PURE CHEMICAL CORPORATION, 049-33583), 1 µM L-3, 30, 5-Triiodothyronine (T3), 100 nM RA and 44 µg/ml vitamin C. S5-2 media: StemFit (registered trademark) Basic03 + 10 µM Alk5i II, 33.3 ng/ml EGF, 10 µM DAPT (N-[N-(3,5-Difluorophenacetyl-L-alanyl)]-S-phenylglycine tert-butyl ester), 1 µM T3, 25 nM RA.
S6 medium: StemFit (registered trademark) Basic03 + 10 µM Alk5i II, 1 µM T3. The inventor conducted 2 conditions as shown in Figure 7B. For M1, in condition a, M1-AKM (IGF1) was used; in condition b, M1-AKM (INS*) (INS*; Apidra insulin that contains no Zn) was used. S2 step was skipped in condition b, and directly switched to S3 medium on day 5.

### (10) A revised protocol for pancreatic differentiation (version 2) (Figures 9 and 10)

The inventor tested two published protocol (Pagliuca et al, 2014, Velazco-Cruz 2019) using the Met, Zn deprival media AKM (IGF1/Zn0.5) or AK03Basic03 [AKM (INS)] for pancreatic differentiation. The media used were as followings.

Initiation of differentiation was done after 5 h complete or methionine deprivation pretreatment, by replacing maintenance media with M1-AKM. On day 3, the media were replaced with S2-AKM. On day 6, replaced with S3-AKM media and cells were cultured for 1 day. Then on day 7 switched to S4-AKM media and cultured for 5 days. Then on day 12, switched to S5 AKM media. On day 19 switched to S6 media.

2 conditions were conducted as shown in Figure 9A. For conditions #1 and #2, medium components are as followings.
S2 AKM medium: AKM (IGF1/Zn0.5) + 50 ng/ml KGF (FUJIFILM Wako Pure Chemical Corporation) and 44 µg/ml vitamin C. S3 AKM medium: StemFit (registered trademark) Basic03 + 50 ng/ml KGF, 0.25 µM SANT, 2 µM Retinoic acid (RA) and 44 µ/ml vitamin C. For condition #1, 500 nM PdBU, 100 nM LDN193189, 10 µM Y27632 were added. For condition #2, 50 nM indolactam V was added, instead.
S4 AKM medium: StemFit (registered trademark) Basic03 + 50 ng/ml KGF, 0.25 µM SANT, 100 nM RA and 44 µg/ml vitamin C. For condition #1, activin A (100 µg/ml) and 10 µM Y27632 were added. For condition #2, 100 nM LDN193189 was added instead.
S5-1 AKM medium: StemFit (registered trademark) Basic03 + 10 µM Alk5i II, 1 µM L-3, 30, 5-Triiodothyronine (T3), 100 nM RA and 44 µg/ml vitamin C. For condition #1, 20 ng/ml betacellulin, 1 µM GSXXi were added. For condition #2, 33.3 ng/ml EGF, 10 µM DAPT were used instead. S5-2 AKM media: StemFit (registered trademark) Basic03 + 10 µM Alk5i II, 1 µM T3, 25 nM RA and 44 µg/ml vitamin C. For condition #1, 20 ng/ml betacellulin, 1 µM GSXXi were added. For condition #2, 33.3 ng/ml EGF and 10 µM DAPT were used instead.

For S6 AKM medium, 6-8 conditions were tested.
a-1: StemFit (registered trademark) Basic03 + 10 µM Alk5i, 1 µM T3.
a-2: StemFit (registered trademark) Basic03 + 10 µM ZnSO₄, Trace Elements A & B.
A-3: AKM (INS*) + 10 µM ZnSO₄.
A-4: AKM (INS*) + 10 µM ZnSO₄, 1 mM NAC, 10 µM Y27632, 50 ng/mL exendin-4, Trace Elements A & B.
b-1: CMRL + fetal bovine serum, 10 µM Alk5i, 1 µM T3. b-2: CMRL + fetal bovine serum, 10 µM ZnSO₄, Trace Elements A & B.
c: Essential 6 + 10 µM Alk5i, 1 µM T3, 10 mM nicotinamide, 1 mM N-acetyl-L-cysteine (NAC), 50 ng/mL exendin-4, 10 µM ZnSO₄.
d: MCDB + 0.046% glucose, bovine serum albumin (BSA), Glutamax, NEAA, 10 µM ZnSO₄, 10 µg/ml heparin, 44 µg/ml vitamin C, Trace Elements A & B.

### (11) Endoderm differentiation of human iPS cells under different Zn concentrations in AKM media containing IGF1, IGF2 or insulin

Undifferentiated Ff-I01s01 cultured on SynthemaxII-coated plate using AK03N were dissociated, and replated onto 6-well suspension culture plates at a density of 5x10⁶ cells/well, and cultured at 37°C for 24 h to form sphere while rotating on a rotating orbital shaker at 95 rpms. Initiation of differentiation was done by replacing AK03N with M1-StemFit (registered trademark) Basic03 or M1-AKM, with IGF1, IGF2 or INS*, supplemented with Zn at different Zn concentration.

The concentrations of Zn used on day 1, day 2 and day 3 are indicated as (x_y_z) µM. For gene expression analysis, on day 3, cells were collected and mRNAs were extracted and subjected for real time PCR analyses using specific primers listed in PrimerArray (registered trademark) Embryonic Stem Cells (ACTB, GAPDH, POU5F1, SOX2, NANOG, CD34, PAX6, GATA4 and GATA6), or made by Takara Bio Inc. in a custom order (GBX2). The primer sequences of GBX2 were as follows: forward primer, CAGGCAATGCCAATTCCAAG (SEQ ID No: 29); reverse primer, CCTGTTCTAGCTGCTGATGCTGAC (SEQ ID No: 30).

### (12) Human pancreatic islets

Non-diabetic human pancreatic islets were obtained from Prodo Laboratories, Inc., and cultured in Prodo Islet Medium (Prodo Laboratories, Inc.). Donor information is as follows. Age, 56; gender, male; ethnicity, Caucasian; body mass index, 13.7; diabetes, no history; and hemoglobin A1c, 5.0%.

### (13) Zn and heavy metal content analysis

Intracellular free and protein bound heavy metal (Zn, Co, Fe, Mg, Ca) contents were measured using High Performance Liquid Chromatography (HPLC) coupled to ICP-MS (Inductively Coupled Plasma -Mass Spectrometry) (HPLC-ICP-MS) in Ajinomoto Co., Inc. as previously described (Arakawa et al, 2016). Contents of heavy metals in the medium were measured before and after chelex100 treatment, then added back. However, Zn was not added in production of ΔZn medium.

### (14) Immunocytochemistry

Cells were fixed in 4% paraformaldehyde (NACALAI TESQUE, INC.) in PBS, permeabilized with 0.1% Triton X-100 (NACALAI TESQUE, INC.), and were then blocked with 20% Blocking One (NACALAI TESQUE, INC., Japan) in PBST (0.1% Tween-20 in PBS). Antibodies were diluted in 20% Blocking One (NACALAI TESQUE, INC., Japan) in PBST (0.1% Tween-20 in PBS). Cells were counterstained with 6-diamidino-2-phenylindole (DAPI) (Roche Diagnostics K.K., Switzerland).

The following primary antibodies were used: mouse anti-Oct3/4 (Santa Cruz Biotechnology, Inc.), goat anti-Sox17 (R&D Systems, Inc.), goat anti-PDX1 (R&D Systems, Inc.), guinea pig anti-insulin (Dako), rabbit anti-C-peptide (Cell Signaling Technology, Inc.), and mouse anti-Nkx6.1 (Developmental Studies Hybridoma Bank). Secondary antibodies used were Alexa 488-conjugated donkey anti-mouse IgG antibodies, Alexa 488-conjugated donkey anti-guinea pig IgG antibodies, Alexa 488-labeled donkey anti-rabbit IgG antibodies, Alexa 568-labeled donkey anti-goat IgG antibodies, and Alexa 568-labeled donkey anti-mouse IgG antibodies (Invitrogen). Positive cells versus total cells (DAPI-positive cells) were quantified using ImageXpress Micro cellular imaging system (Molecular Devices, LLC.).

### (15) Real time RT-PCR analysis

RNA was extracted from iPS cells using the RNeasy micro-kit, or All prep (DNA/RNA) Micro Kit (QIAGEN K.K., Germany) and then treated with DNase (QIAGEN K.K.). For reverse transcription reactions, 1 µg RNA was reverse-transcribed using PrimeScript (registered trademark) RT Master Mix (Takara Bio Inc., Japan). For real-time PCR analysis, the mRNA expression was quantified with SyberGreen on a StepOne Plus (Applied Biosystems, Foster City, CA). The PCR conditions were as follows: Initial denaturation at 95°C for 30 sec, followed with denaturation at 95°C for 5 sec, annealing and extension at 60°C for 30 sec, for up to 40 cycles. Target mRNA levels were expressed as arbitrary units and were determined using the ΔΔ CT method.

PrimerArray Embryonic Stem Cells (Human) (TaKaRa Bio Inc., #PH016, Kyoto) containing primer pairs optimized for real-time RT-PCR analysis of 88 genes associated with pluripotency and self-renewal of embryonic stem cells and 8 housekeeping genes, were used.

Primer sequences for SLC30A and SLC39A genes are listed in the following table and SEQ ID Nos: 1 to 28.

**[Table 1]**

| Gene | Forwad | Reverse |
|---|---|---|
| *GAPDH* | GCACCGTCAAGGCTGAGAAC | TGGTGAAGACGCCAGTGGA |
| *SLC30A1* | CACCAGGAGGAGACCAACAC | TCACCACTTCTGGGGTTTTC |
| *SLC30A5* | TTTCAGCATAGCTCTCAATCGATCC | GCACGCCATAGAATAATTCCACAA |
| *SLC30A6* | TTGGAACAGCCCGAGATACACA | CATCGTGAACAGGTTGAAACAAAGA |
| *SLC30A7* | GCTGGACTGGCAGCTTCTGTTA | CCAGCCAGAACTTCCGCTCTA |
| *SLC30A9* | GCCACTTGCATGGGCCTTAC | CTGGATGGACCGCCCTAAGA |
| *SLC39A1* | CCTGACTACCTGGCTGCCATA | GGATGAACTCTTGCAGTGGGAAC |
| *SLC39A6* | GGTGATGGCCTGCACAATTTC | TTAACGGTCATGCCAGCCTTTAG |
| *SLC39A7* | ACTTTGCCATCTTGGTCCAGTC | ACGTAGATAAAGCCACCTGCAGTAA |
| *SLC39A8* | CCTAAAGCATTACCTGCCATCAA | TCGCAGAGCGTTATCATCCA |
| *SLC39A9* | ATGTTACGTGGCCGGAATCA | TGCCAGAGCAGTTCCACAGAG |
| *SLC39A10* | TCTGTCATGAACTGCCACATGAA | CATACTGACCAACAGCTGTGCCTA |
| *SLC39A11* | GGGCAGCTCTCGTGTTCGTA | GCAGCAAAGCCAAGACTTCCA |
| *SLC39A14* | CTACTTCATAGCTCTGGCGATTGGA | CACCACTGCAGACTTGGAGAGATAA |

### (16) Affymetrix Microarray analysis

Human iPSC-derived differentiated cells on differentiation day 3 DE cells, differentiation day 5 PG cells, differentiation day 11 PP cells, and differentiation day 25 EC cells were compared, using GeneChip (registered trademark) Human Gene 2.0 ST Array (Applied Biosystems). Expression profile of undifferentiated human ESCs (KhES3), or 201B7 hiPSCs cultured under methionine deprivation or control complete medium were compared using GeneChip (registered trademark) Human Genome U133 Plus 2.0 Array (Applied Biosystems), GeneChip (registered trademark) Human Gene 2.0 ST Array (Applied Biosystems), or Human Transcriptome Array (HTA) 2.0 (Applied Biosystems). Comparison #1 201B7_CSTI7_ST Array and #2 khES3_CSTI7_U133 Array (GSE55285): cells were cultured on matrigel-coated dish with ReproFF (ReproCELL Inc.) and cultured in control complete CSTI-7 medium (Cell Science & Technology Institute, Inc.) or Met-deprived CSTI-7 medium for 5h. Comparison #3 201B7_StemFit _HTA: cells were cultured on SynthemaxII-coated dish with control complete AK02N or Met-deprived StemFit media (KA01).

### (17) Principal component analysis

A Princical Component Analysis (PCA) was performed, to visualize the results and map the expression profile among undifferentiated cells, hiPSC-derived differentiated cells on differentiation day 3 DE cells, differentiation day 5 PG cells, differentiation day 11 PP cells, and differentiation day 25 EC cells (Figure 1C). Figure 2D shows PCA analysis using 88 genes associated with pluripotency and self-renewal of embryonic stem cells and 8 housekeeping genes, among undifferentiated cells grown in amino acids singly deprived media for 5 hrs. PCA was performed using a JMP software (SAS Institute Inc., Japan).

### (18) C-peptide release and content assay

The C-peptide release and content assay was performed as described previously (Sakano et al., 2014) with minor modifications. Briefly, differentiated cells were pre-incubated at 37°C for 2 h with low glucose media, DMEM (Life Technologies) containing 3 mM glucose and 0.2% BSA. Cells were washed twice with PBS and then incubated at 37°C for 1 h with low glucose medium. The culture medium was collected, and the same cells were further incubated with high glucose medium, DMEM containing 20 mM glucose and 0.2% BSA, at 37°C for another 1 h. The culture solution was collected and stored at -80°C until analysis. Next, the cells were lysed and purified both RNA and DNA using AllPrep DNA/RNA Micro Kit (QIAGEN K.K.).

C-peptide secretion into the culture media were measured using the human C-peptide ELISA Kit (FUJIFILM Wako Shibayagi Corporation, Japan). The amount of C-peptide was normalized to the amount of total DNA contents in the corresponding cell lysate.

### (19) New GSIS method

Differentiated cells were loaded on Transwell (Corning Incorporated, #3415) and then pre-incubated 4 x 10 min + 30 min at 37°C in low glucose (3 mM) HKRB buffer (HEPES Krebs-Ringer Bicarbonate buffer, Cosmo Bio Co., Ltd.) with 0.2% BSA, on an orbital shaker (70 rpm). The buffer was collected (low glucose), and the same cells were further incubated in high glucose (20 mM) HKRB buffer with 0.2% BSA, at 37°C on an orbital shaker (70 rpm) for another 1 h. During 1 h incubation in high glucose buffer, 10% of supernatant was collected at 10 and 30 min, and then added back same volumes of high glucose buffer. Collected culture supernatant was stored at -30°C until analysis. Next, cells were lysed, and RNA and DNA were purified using AllPrep DNA/RNA Micro Kit (QIAGEN K.K.). C-peptide secretion was measured using a human C-peptide ELISA Kit (Mercodia AB). The amount of C-peptide was normalized to the amount of total DNA contents in the corresponding cell lysate.

### (20) Statistics

Data are expressed as the mean ± SEM. Differences between groups were analyzed by Student's t-tests or one-way ANOVA and Dunnett's multiple comparisons test. The statistical analyses and P-values thereof are noted in legends of their respective figures. *p < 0.05, **p < 0.01 or ***p < 0.005 by Student's t-test; ^{§}P < 0.05 or ^{§§}P < 0.01, by one-way ANOVA and Dunnett's multiple comparisons test, are considered to be significant.

### [References]

Andrews, G.K., Wang, H., Dey, S.K., and Palmiter, R.D. (2004). Mouse zinc transporter 1 gene provides an essential function during early embryonic development. Genesis 40, 74-81.
Mitsui, K., Tokuzawa, Y., Itoh, H., Segawa, K., Murakami, M., Takahashi, K., Maruyama, M., Maeda, M., and Yamanaka, S. (2003). The homeoprotein Nanog is required for maintenance of pluripotency in mouse epiblast and ES cells. Cell 113, 631-642.
Nakashima, R., Morooka, M., Shiraki, N., Sakano, D., Ogaki, S., Kume, K., and Kume, S. (2015). Neural cells play an inhibitory role in pancreatic differentiation of pluripotent stem cells. Genes Cells 1028-1045.
Shahjalal, H.M., Shiraki, N., Sakano, D., Kikawa, K., Ogaki, S., Baba, H., Kume, K., and Kume, S. (2014). Generation of insulin-producing β-like cells from human iPS cells in a defined and completely xeno-free culture system. J. Mol. Cell Biol. 0, 1-15.
Shi, Z.D., Lee, K., Yang, D., Amin, S., Verma, N., Li, Q. V., Zhu, Z., Soh, C.L., Kumar, R., Evans, T., et al. (2017). Genome Editing in hPSCs Reveals GATA6 Haploinsufficiency and a Genetic Interaction with GATA4 in Human Pancreatic Development. Cell Stem Cell 20, 675-688.e6.
Sugita, S., Iwasaki, Y., Makabe, K., Kimura, T., Futagami, T., Suegami, S., and Takahashi, M. (2016). Lack of T Cell Response to iPSC-Derived Retinal Pigment Epithelial Cells from HLA Homozygous Donors. Stem Cell Reports 7, 619-634.
Takahashi, K., and Yamanaka, S. (2016). A decade of transcription factor-mediated reprogramming to pluripotency. Nat. Rev. Mol. Cell Biol. 17, 183-193. Yoshioka, N., Gros, E., Li, H.R., Kumar, S., Deacon, D.C., Maron, C., Muotri, A.R., Chi, N.C., Fu, X.D., Yu, B.D., et al. (2013). Efficient generation of human iPSCs by a synthetic self-replicative RNA. Cell Stem Cell 13, 246-254.
Zhu, S., Russ, H.A., Wang, X., Zhang, M., Ma, T., Xu, T., Tang, S., Hebrok, M., and Ding, S. (2016). Human pancreatic beta-like cells converted from fibroblasts. Nat. Commun. 7, 10080.

All publications, patents, and patent applications cited in this specification are incorporated herein by reference as they are.

### Industrial Applicability

The present invention is a method for promoting differentiation of pluripotent stem cells into cells of pancreas, liver, intestine, and the like and thus can be used in the industrial field using such cells.

## Claims

1. A method for promoting differentiation of pluripotent stem cells, comprising: a step of culturing pluripotent stem cells in a medium, wherein the medium is a medium comprising a) an insulin-like growth factor, b) an insulin analogue preparation containing no zinc, c) an insulin analogue preparation containing a low concentration of zinc, or d) a compound exhibiting an insulin-like action.

2. The method for promoting differentiation of pluripotent stem cells according to claim 1, wherein the medium for culturing pluripotent stem cells is a medium comprising no insulin.

3. The method for promoting differentiation of pluripotent stem cells according to claim 1 or 2, wherein the medium for culturing pluripotent stem cells is a medium comprising zinc at a concentration of 1 µM or less or a medium comprising no zinc.

4. The method for promoting differentiation of pluripotent stem cells according to any one of claims 1 to 3, wherein the medium for culturing pluripotent stem cells is a medium allowing the pluripotent stem cells to differentiate into endoderm cells.

5. The method for promoting differentiation of pluripotent stem cells according to any one of claims 1 to 4, comprising: a step of culturing pluripotent stem cells in an undifferentiated maintenance medium, wherein the undifferentiated maintenance medium is a medium comprising no methionine.

6. The method for promoting differentiation of pluripotent stem cells according to claim 1, wherein the medium for culturing pluripotent stem cells is a medium comprising an activin receptor-like kinase-4,7 activator and comprising zinc at a concentration of 1 µM or less or no zinc.

7. The method for promoting differentiation of pluripotent stem cells according to claim 6, wherein the activin receptor-like kinase-4,7 activator is human activin A, and a concentration thereof is 6 to 150 ng/mL.

8. A medium for culturing pluripotent stem cells, comprising: a) an insulin-like growth factor, b) an insulin analogue preparation comprising no zinc, c) an insulin analogue preparation comprising a low concentration of zinc, or d) a compound exhibiting an insulin-like action.

9. The medium according to claim 8, comprising no insulin.

10. The medium according to claim 8 or 9, comprising zinc at a concentration of 1 µM or less or no zinc.

11. The medium according to any one of claims 8 to 10, wherein the medium is a medium allowing the pluripotent stem cells to differentiate into endoderm cells.

12. The medium according to claim 8, wherein the medium for culturing pluripotent stem cells is a medium comprising an activin receptor-like kinase-4,7 activator and comprises zinc at a concentration of 1 µM or less or no zinc.

13. The medium according to claim 12, wherein the activin receptor-like kinase-4,7 activator is human activin A, and a concentration thereof is 6 to 150 ng/mL.

14. A method for inducing differentiation of pluripotent stem cells into insulin-producing cells, comprising steps (1) to (6) below:
(1) a step of culturing pluripotent stem cells in a medium comprising no methionine;
(2) a step of culturing the cells obtained in step (1) in the medium according to claim 12 or 13;
(3) a step of culturing the cells obtained in step (2) in a medium comprising an FGF and a hedgehog signaling inhibitor;
(4) a step of culturing the cells obtained in step (3) in a medium comprising a retinoic acid receptor agonist, a hedgehog signaling inhibitor, and a BMP signaling inhibitor;
(5) a step of culturing the cells obtained in step (4) in a medium comprising a TGF-βI type activin receptor-like kinase-4,5,7 inhibitor, a protein kinase C activator, and a BMP signaling inhibitor; and
(6) a step of culturing the cells obtained in step (5) in a medium comprising nicotinamide.

15. The method for inducing differentiation into insulin-producing cells according to claim 14, wherein each medium in step (1) to (6) is a xeno-free medium.

16. A method for inducing differentiation of pluripotent stem cells into insulin-producing cells, comprising steps (1) to (9), or (1) to (3) and (5) to (9) below:
(1) a step of culturing pluripotent stem cells in a medium comprising no methionine;
(2) a step of culturing the cells obtained in step (1) in the medium according to claim 12 or 13;
(3) a step of culturing the cells obtained in step (2) in a medium comprising an FGF and a hedgehog signaling inhibitor;
(4) a step of culturing the cells obtained in step (3) in a medium comprising a KGF;
(5) a step of culturing the cells obtained in step (3) or step (4) in a medium comprising a KGF, a hedgehog signaling inhibitor, a protein kinase C activator, and a retinoic acid receptor agonist;
(6) a step of culturing the cells obtained in step (5) in a medium comprising a KGF, a hedgehog signaling inhibitor, a retinoic acid receptor agonist, and a BMP signaling inhibitor;
(7) a step of culturing the cells obtained in step (6) in a medium comprising a TGF-βI type activin receptor-like kinase-4,5,7 inhibitor, an EGF receptor agonist, a γ-secretase inhibitor, and a retinoic acid receptor agonist;
(8) a step of culturing the cells obtained in step (7) in a medium comprising a TGF-βI type activin receptor-like kinase-4,5,7 inhibitor, an EGF receptor agonist, a γ-secretase inhibitor, and a retinoic acid receptor agonist, wherein a concentration of the retinoic acid receptor agonist in the medium is lower than a concentration of the retinoic acid receptor agonist in step (7); and
(9) a step of culturing the cells obtained in step (8) in a medium comprising a TGF-βI type activin receptor-like kinase-4,5,7 inhibitor.

17. A method for differentiating pancreatic progenitor cells into pancreatic beta cells, comprising culturing pancreatic progenitor cells in a medium comprising a factor selected from the group consisting of a retinoic acid receptor agonist, a hedgehog signaling inhibitor, a KGF, a γ-secretase inhibitor, a BMP signaling inhibitor, a TGF-βI type activin receptor-like kinase-4,5,7 inhibitor, and an EGF receptor agonist, wherein the pancreatic progenitor cells are PDX1 positive or NKX6.1 positive.

18. A method for inducing differentiation of pluripotent stem cells into insulin-producing cells, comprising steps (1) to (7) below:
(1) a step of culturing pluripotent stem cells in a medium comprising no methionine;
(2) a step of culturing the cells obtained in step (1) in the medium according to claim 12 or 13;
(3) a step of culturing the cells obtained in step (2) in a medium comprising a KGF and comprising zinc at a concentration of 1 µM or less or no zinc;
(4) a step of culturing the cells obtained in step (3) in a medium comprising a KGF, a hedgehog signaling inhibitor, and a retinoic acid receptor agonist;
(5) a step of culturing the cells obtained in step (4) in a medium comprising a KGF, a hedgehog signaling inhibitor, and a retinoic acid receptor agonist, wherein a concentration of the retinoic acid receptor agonist in the medium is lower than a concentration of the retinoic acid receptor agonist in step (4);
(6) a step of culturing the cells obtained in step (5) in a medium comprising a TGF-βI type activin receptor-like kinase-4,5,7 inhibitor and a retinoic acid receptor agonist; and
(7) a step of culturing the cells obtained in step (6) in a medium comprising a TGF-βI type activin receptor-like kinase-4,5,7 inhibitor and a retinoic acid receptor agonist, wherein a concentration of the retinoic acid receptor agonist in the medium is lower than a concentration of the retinoic acid receptor agonist in step (6).


SEQUENCE LISTING

<110> Tokyo Institute of Technology
      Ajinomoto Co., Inc.

<120> METHOD FOR PROMOTING THE DIFFERENTIATION OF PLURIPOTENT STEM CELLS

<130> FP-257PCT

<150> JP 2018/085591
<151> 2018-4-26

<150> JP 2018/232436
<151> 2018-12-12

<160> 30

<170> PatentIn Ver. 2.1

<210> 1
<211> 20
<212> DNA
<213> Artificial Sequence

<220>
<223> Description of Artificial Sequence:primer

<400> 1
gcaccgtcaa ggctgagaac                                              20


<210> 2
<211> 19
<212> DNA
<213> Artificial Sequence

<220>
<223> Description of Artificial Sequence:primer

<400> 2
tggtgaagac gccagtgga                                               19


<210> 3
<211> 20
<212> DNA
<213> Artificial Sequence

<220>
<223> Description of Artificial Sequence:primer

<400> 3
caccaggagg agaccaacac                                              20


<210> 4
<211> 20
<212> DNA
<213> Artificial Sequence

<220>
<223> Description of Artificial Sequence:primer

<400> 4
tcaccacttc tggggttttc                                                    20

<210> 5
<211> 25
<212> DNA
<213> Artificial Sequence

<220>
<223> Description of Artificial Sequence:primer

<400> 5
tttcagcata gctctcaatc gatcc                                              25

<210> 6
<211> 24
<212> DNA
<213> Artificial Sequence

<220>
<223> Description of Artificial Sequence:primer

<400> 6
gcacgccata gaataattcc acaa                                               24

<210> 7
<211> 22
<212> DNA
<213> Artificial Sequence

<220>
<223> Description of Artificial Sequence:primer

<400> 7
ttggaacagc ccgagataca ca                                                 22

<210> 8
<211> 25
<212> DNA
<213> Artificial Sequence

<220>
<223> Description of Artificial Sequence:primer

<400> 8
catcgtgaac aggttgaaac aaaga                                              25

<210> 9
<211> 22
<212> DNA
<213> Artificial Sequence

<220>
<223> Description of Artificial Sequence:primer

<400> 9
gctggactgg cagcttctgt ta                                                 22

<210> 10
<211> 21
<212> DNA
<213> Artificial Sequence

<220>
<223> Description of Artificial Sequence:primer

<400> 10
ccagccagaa cttccgctct a                                                21


<210> 11
<211> 20
<212> DNA
<213> Artificial Sequence

<220>
<223> Description of Artificial Sequence:primer

<400> 11
gccacttgca tgggccttac                                                  20


<210> 12
<211> 20
<212> DNA
<213> Artificial Sequence

<220>
<223> Description of Artificial Sequence:primer

<400> 12
ctggatggac cgccctaaga                                                  20


<210> 13
<211> 21
<212> DNA
<213> Artificial Sequence

<220>
<223> Description of Artificial Sequence:primer

<400> 13
cctgactacc tggctgccat a                                                21


<210> 14
<211> 23
<212> DNA
<213> Artificial Sequence

<220>
<223> Description of Artificial Sequence:primer

<400> 14
ggatgaactc ttgcagtggg aac                                              23


<210> 15
<211> 21

<212> DNA
<213> Artificial Sequence

<220>
<223> Description of Artificial Sequence:primer

<400> 15
ggtgatggcc tgcacaattt c                                                21


<210> 16
<211> 23
<212> DNA
<213> Artificial Sequence

<220>
<223> Description of Artificial Sequence:primer

<400> 16
ttaacggtca tgccagcctt tag                                              23


<210> 17
<211> 22
<212> DNA
<213> Artificial Sequence

<220>
<223> Description of Artificial Sequence:primer

<400> 17
actttgccat cttggtccag tc                                               22


<210> 18
<211> 25
<212> DNA
<213> Artificial Sequence

<220>
<223> Description of Artificial Sequence:primer

<400> 18
acgtagataa agccacctgc agtaa                                            25


<210> 19
<211> 23
<212> DNA
<213> Artificial Sequence

<220>
<223> Description of Artificial Sequence:primer

<400> 19
cctaaagcat tacctgccat caa                                              23


<210> 20
<211> 20
<212> DNA
<213> Artificial Sequence

<220>
<223> Description of Artificial Sequence:primer

<400> 20
tcgcagagcg ttatcatcca                                                    20


<210> 21
<211> 20
<212> DNA
<213> Artificial Sequence

<220>
<223> Description of Artificial Sequence:primer

<400> 21
atgttacgtg gccggaatca                                                    20


<210> 22
<211> 21
<212> DNA
<213> Artificial Sequence

<220>
<223> Description of Artificial Sequence:primer

<400> 22
tgccagagca gttccacaga g                                                  21


<210> 23
<211> 23
<212> DNA
<213> Artificial Sequence

<220>
<223> Description of Artificial Sequence:primer

<400> 23
tctgtcatga actgccacat gaa                                                23


<210> 24
<211> 24
<212> DNA
<213> Artificial Sequence

<220>
<223> Description of Artificial Sequence:primer

<400> 24
catactgacc aacagctgtg ccta                                               24


<210> 25
<211> 20
<212> DNA
<213> Artificial Sequence

<220>
<223> Description of Artificial Sequence:primer

<400> 25
gggcagctct cgtgttcgta                                                    20


<210> 26
<211> 21
<212> DNA
<213> Artificial Sequence

<220>
<223> Description of Artificial Sequence:primer

<400> 26
gcagcaaagc caagacttcc a                                                  21


<210> 27
<211> 25
<212> DNA
<213> Artificial Sequence

<220>
<223> Description of Artificial Sequence:primer

<400> 27
ctacttcata gctctggcga ttgga                                              25


<210> 28
<211> 25
<212> DNA
<213> Artificial Sequence

<220>
<223> Description of Artificial Sequence:primer

<400> 28
caccactgca gacttggaga gataa                                              25


<210> 29
<211> 20
<212> DNA
<213> Artificial Sequence

<220>
<223> Description of Artificial Sequence:primer

<400> 29
caggcaatgc caattccaag                                                    20


<210> 30
<211> 24
<212> DNA
<213> Artificial Sequence

<220>
<223> Description of Artificial Sequence:primer

<400> 30
cctgttctag ctgctgatgc tgac                                               24